(19) Europäisches Patentamt
European Patent Office
Office européen des brevets

(11) **EP 3 657 236 B1**

(12) **EUROPÄISCHE PATENTSCHRIFT**

(45) Veröffentlichungstag und Bekanntmachung des Hinweises auf die Patenterteilung:
**16.12.2020 Patentblatt 2020/51**

(21) Anmeldenummer: **19215546.3**

(22) Anmeldetag: **01.06.2018**

(51) Int Cl.:
*G02C 13/00* (2006.01)    *G06Q 30/06* (2012.01)
*G06T 17/00* (2006.01)    *G06T 19/20* (2011.01)
*A61B 5/107* (2006.01)    *G06T 19/00* (2011.01)
*G06T 7/10* (2017.01)    *G06T 7/33* (2017.01)
*G16H 20/40* (2018.01)    *G16H 30/40* (2018.01)
*G16H 40/63* (2018.01)

(54) **VERFAHREN, VORRICHTUNG UND COMPUTERPROGRAMM ZUM VIRTUELLEN ANPASSEN EINER BRILLENFASSUNG**

METHOD, DEVICE AND COMPUTER PROGRAM FOR VIRTUAL ADAPTING OF A SPECTACLE FRAME

PROCÉDÉ, DISPOSITIF ET PROGRAMME INFORMATIQUE DESTINÉS À L'ADAPTATION VIRTUELLE D'UNE MONTURE DE LUNETTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.06.2017 EP 17173929**

(43) Veröffentlichungstag der Anmeldung:
**27.05.2020 Patentblatt 2020/22**

(62) Dokumentnummer(n) der früheren Anmeldung(en) nach Art. 76 EPÜ:
**18727024.4 / 3 631 570**

(73) Patentinhaber: **Carl Zeiss Vision International GmbH**
**73430 Aalen (DE)**

(72) Erfinder:
• SCHWARZ, Oliver
  73479 Ellwangen (DE)
• IHRKE, Ivo
  73486 Adelmannsfelden (DE)

(74) Vertreter: **Sticht, Andreas**
**Kraus & Weisert**
**Patentanwälte PartGmbB**
**Thomas-Wimmer-Ring 15**
**80539 München (DE)**

(56) Entgegenhaltungen:
US-A1- 2010 022 304    US-A1- 2013 088 490
US-A1- 2016 246 078

**Beschreibung**

Die vorliegende Anmeldung betrifft Verfahren, Vorrichtungen und Computerprogramme zum virtuellen Anpassen von Brillenfassungen. Unter einer Brillenfassung ist dabei in Übereinstimmung mit DIN EN ISO 7998:2006-01 und DIN EN ISO 8624:2015-12 ein Gestell oder eine Halterung zu verstehen, mittels dem/der Brillengläser am Kopf getragen werden können. Der Begriff wie hier verwendet beinhaltet insbesondere auch randlose Brillenfassungen. Derartige Brillenfassungen werden umgangssprachlich auch als Brillengestelle bezeichnet. Ein virtuelles Aufsetzen einer Brillenfassung bezeichnet im Rahmen der vorliegenden Anmeldung ein Anpassen eines Modells einer Brillenfassung an ein Modell eines Kopfes auf einer Recheneinrichtung, üblicherweise verbunden mit einer graphischen Darstellung des Anpassens der Brillenfassung auf einen Kopf einer Person auf einer Anzeige, beispielsweise einem Computerbildschirm.

Ein virtuelles Aufsetzen einer Brillenfassung ist beispielsweise aus der US 2003/0123026 A1 oder der US 2002/105530 A1 bekannt. Bei diesen Druckschriften dient das virtuelle Aufsetzen der Brillenfassung hauptsächlich dazu, einem Benutzer die Auswahl zwischen verschiedenen Brillenfassungen zu erleichtern, indem eine graphische Darstellung des Kopfes des Benutzers zusammen mit der Brillenfassung angezeigt wird.

Die WO 2015/101738 A2 entsprechend der US 2016/0327811 A1 offenbart ein Verfahren zur virtuellen Anpassung einer Brillenfassung an einen Benutzer. Hierbei wird ein Modell des Kopfes des Benutzers, insbesondere ein Modell der Nase und ein Modell der beiden Ohren des Benutzers verwendet. Dabei wird auf Basis einer Überlagerung eines Modells der Brillenfassung mit dem Modell des Kopfes eine benötigte Deformation der Brillenfassung bestimmt, indem ein überlappender Bereich minimiert wird, sodass die Brillenfassung auf den Kopf des Benutzers passt. Diese Deformation kann dann durch eine Maschine oder auch manuell auf die entsprechende reale Brillenfassung angewendet werden. Zusätzlich kann bei dieser Druckschrift eine Position des Brillengestells bezüglich der Nase automatisch optimiert werden. Diese Optimierung ist relativ rechenaufwändig, und je nach Startwerten der Optimierung ist nicht unbedingt sichergestellt, dass tatsächlich eine optimale Anpassung erfolgt.

Die US 9,286,715 B2 offenbart ein Verfahren zur virtuellen Anprobe einer Brille. Dabei werden sowohl auf eine Brillenfassung als auch auf einem Kopf mehrere Punkte definiert. Eine Positionierung der Brillenfassung am Kopf erfolgt, indem ausgewählte Punkte auf der Brillenfassung mit ausgewählten Punkten auf dem Kopf in Übereinstimmung gebracht werden. Eine Positionsänderung erfolgt durch Ändern der ausgewählten Punkte. Dies ermöglicht eine Positionierung mit einer Genauigkeit, die für den Zweck der US 9,286,715 B2, eine virtuelle Anprobe zur Erlangung eines visuellen Eindrucks zu erlangen, ausreichend ist. Eine genaue Anpassung, auf Basis derer ein Optiker dann eine reale Anpassung eines Brillengestells vornehmen kann, wird hier nicht erreicht.

Von dem Unternehmen Volumental ist ein Demonstrationsvideo der Software "Vacker" auf "https://www.volumental.com/face-scanning/", Stand 5. März 2017, verfügbar, bei welcher ein Kopf mit einer aufgesetzten Brille dargestellt ist und Parameter der Brille mittels Schiebereglern modifizierbar sind, beispielsweise des Sitzes der Brille auf dem Nasenrücken oder auch andere Parameter wie Fassungsscheibenwinkel und Vorneigung (vgl. DIN EN ISO). Auch eine Farbe der Brillenfassung oder eine Farbe der Scharniere der Brillenfassung können ausgewählt werden.

Videos, die einen Workflow einer virtuellen Brillenanpassung zeigen, sind auf dem Webportal von Alain Afflelou/Ditto verfügbar, vergleiche beispielsweise "https://www.youtube.com/watch?v=awNs2cEoZ7Y" oder "https://www.youtube.com/watch?v=ZsBV4FkcU8U", Stand 5. März 2017. Dabei wird ein Computer mit integrierter Kamera als Aufnahmesystem genutzt, und es können Fassungen ausgewählt werden. Bei dieser Herangehensweise erfolgt die Brillenanpassung gleichsam auf einem Livevideo des Benutzers, welches über die Kamera aufgenommen wird.

Der oben erläuterte Stand der Technik erlaubt ein virtuelles Aufsetzen einer Brille und beispielsweise bei der WO 2015/101738 A2 bis zu einem gewissen Grad auch ein Anpassen der Brille an den Benutzer, um diese Anpassung dann auf eine reale Brillenfassung zu übertragen, sodass dem Benutzer anschließend direkt mit möglichst geringen weiteren Anpassungen eine Brille mit der Brillenfassung bereitgestellt werden kann. Wie erläutert ist das Verfahren der WO 2015/101738 A2 aber vergleichsweise rechenaufwändig, da eine Optimierung anhand der Überlagerung von 3D-Modellen durchgeführt wird. Zudem ist es bei dem Optimierungsverfahren dieser Druckschrift unsicher, ob tatsächlich ein Optimum gefunden wird, oder ob das Optimierungsverfahren z.B. in einem lokalen Minimum einer zu optimierenden Größe "hängen bleibt".

Die US 2016/360970 A1 offenbart tragbare Vorrichtungen zur Durchführung thermischer Messungen, die insbesondere Wärmebildkameras enthalten, die an einem Brillengestell angebracht sind.

Hossein-Nejad Zahra et al.: "RKEM: Redundant Keypoint Elimination Method in Image Registration", IET Image Processing, IET, UK, Bd. 11, Nr. 5, 1. Mai 2017 (2017-05-01), Seiten 273-284, XP006061590, ISSN: 1751-9659, DOI: 10.1049/IET-IPR.2016.0440 betrifft die Identifikation von Bildmerkmalen unter Benutzung von Schlüsselpunkten (Keypoints) und Algorithmen, um derartige Schlüsselpunkte zu bestimmen.

Die US 2016/0246078 A1 offenbart die Erzeugung eines realistisch aussehenden Bildes einer Brille, die auf einem Bild einer Person positioniert ist. Hierzu wird das Gesicht analysiert, um eine Rotationsmatrix, einen Translationsvektor und Morphologieparameter des Gesichts des Benutzers zu erhalten.

Die US 2010/0022304 A1 offenbart ein Touchpad für Spielanwendungen, wobei es darum geht, eine Spielfigur zur bewegen.

Die US 2015/0055085 A1 offenbart eine Vielzahl von Verfahren und Möglichkeiten, eine virtuelle Brillenfassung einem Kopfmodell anzupassen.

Die WO 2013/177456 A1 offenbart ein Verfahren zur virtuellen Brillenanpassung, welche Punkte auf einem Nasenrücken und Punkte bei den Ohren eines Kopfes benutzt.

Die DE 102 16 824 A1 betrifft Verfahren und Vorrichtungen zum Konstruieren einer Maßbrille unter Benutzung einer Computervorrichtung.

Ein weiteres Verfahren zum Personalisieren von Brillenfassungen ist aus der FR 3 065 295 A1 bekannt.

Die US 2013 088490 A1 offenbart ein Verfahren gemäß dem Oberbegriff von Anspruch 1.

Ein weiteres Verfahren zur virtuellen Brillenanpassung ist aus der FR 301 6051 A1 bekannt.

Die ES 260 4806 A2 offenbart Verfahren zur Bestimmung von Parametern für Gläser von Brillengestelle und zur Bestellung von so bestimmten Gläsern. Die beschriebenen Verfahren umfassen: Erhalten morphologischer Daten von dem Kopf des Benutzers, Bestimmen der optimalen Position der Linsen, Bestimmen der möglichen Brillengestelle aus den Brillengestellen einer Brillengestelldatenbank und Ausschließen der nicht möglichen Brillengestelle, Berechnen eines möglichen Paars von Linsen für jedes mögliche Brillengestell und Ausschließen von Brillengestellen ohne brauchbare Linsen, Auswählen mindestens eines realisierbaren Brillengestells aus einer Datenbank realisierbarer Gläser durch einen Benutzer, Bestätigen einer Auswahl und Platzieren einer Bestellung.

Die WO 01/88654 A2 offenbart ein Brillenanpassungssystem mit einem Bildgebungssystem, das eine Sicht auf das Gesicht eines Kunden ermöglicht, einem 3D-Bildprozessor zum Erzeugen von ersten 3D-Informationen, die die physischen Merkmale des Kunden beschreiben, einer virtuellen Anprobeeinheit zum Empfangen der digitalen 3D-Darstellung des Gesichts des Kunden und eine digitale 3D-Darstellung eines Brillengestells, um das Brillengestell virtuell auf dem Gesicht des Kunden zu aufzusetzen und eine zweite 3D-Information zu erzeugen, die Rahmen-/Gesichtspassung beschreibt, und einen Linsenanpasser, der eingerichtet ist, die ersten 3D-Informationen und die zweiten 3D-Information zu empfangen und daraus mindestens einen Parameter zur kundenspezifischen Gesichts- und Rahmenherstellung und zum Schleifen der Linse zu erzeugen. In diesem Zusammenhang offenbart die WO 01/88654 A2 ein automatisches Zuordnen von Gesichtsmerkmalen mittels einem "3D face extractor" sowie die manuelle Bestimmung von Kopf-Metadaten mittels manueller Vermessung.

Die WO 2016 164859 A1 offenbart Systeme und Verfahren zum Erzeugen einer Brillenrahmen- und Linsengeometrie, die an die Anatomie eines Benutzers angepasst und für den Brechwert des Benutzers optimiert ist. Ein Verfahren umfasst: Empfangen eines konfigurierbaren parametrischen Modells eines benutzerspezifischen Brillenprodukts, das einen Rahmenteil und einen Linsenteil umfasst, wobei geometrische Parameter des konfigurierbaren parametrischen Modells auf geometrischen Merkmalen der Anatomie eines Benutzers basieren; Empfangen von Mediendaten eines Benutzers, wobei die Mediendaten die Reaktion des Benutzers auf visuelle Hinweise enthalten; Erfassen der Position der Augen des Benutzers aus den empfangenen Mediendaten; Bestimmen der optischen Information des Benutzers basierend auf der erkannten Position der Augen des Benutzers; und Erzeugen eines aktualisierten konfigurierbaren parametrischen Models durch Modifizieren des empfangenen konfigurierbaren parametrischen Modells basierend auf der bestimmten optischen Information. In diesem Zusammenhang offenbart die WO 2016 164859 A1 die Verwendung von maschinellem Lernen zur Detektion von Gesichtsmerkmalen und die Berechnung von Informationen zur Beleuchtung bei Aufnahmen eines Kopfes sowie die virtuelle Darstellung von Brillengestellen mit entsprechend angepasster virtueller Beleuchtung.

Mittels der in FR 301 6051 A1 beschriebenen Verfahren ist es möglich, eine Brillenfassung virtuell anzuprobieren, wobei die Positionierung der Brillenfassung auf dem Kopf des Nutzers mittels einer Grob- und einer Feinpositionierung erfolgt.

Aus der US 2013 088490 A1 ist eine Segmentierung des 3D-Modells der Brillenfassung in Komponenten der Brillenfassung bekannt. Ausgehend von US 2013 088490 A1 ist es eine Aufgabe der vorliegenden Anmeldung, die Segmentierung rechnerisch einfacher implementierbar zu machen. Hierzu wird ein Verfahren gemäß Anspruch 1 bereitgestellt.

Die Unteransprüche definieren weitere Ausführungsbeispiele.

Ebenfalls wird ein Computerprogramm gemäß Anspruch 6 und Vorrichtungen gemäß Anspruch 7 bereitgestellt.

Die Erfindung wird nachfolgend kurz diskutiert. Verwendete Begriffe werden nach dieser kurzen Diskussion definiert.

Erfindungsgemäß wird ein computerimplementiertes Verfahren zur virtuellen Brillenanpassung bereitgestellt, umfassend:

virtuelles Feinpositionieren einer Brillenfassung auf Basis eines 3D-Modells (30, 31) eines Kopfes und eines 3D-Modells (40) der Brillenfassung,

virtuelles Grobpositionieren der Brillenfassung auf dem Kopf auf Basis von Kopf-Metadaten für das 3D-Modell (30,31) des Kopfes und von Fassungs-Metadaten für das 3D-Modell der Brillenfassung vor dem virtuellen Feinpositionieren,
wobei das Bereitstellen der Fassungs-Metadaten eine Segmentierung des 3D-Modells der Brillenfassung in Komponenten der Brillenfassung umfasst, wobei die Komponenten bevorzugt mindestens eine Komponente aus der Gruppe

- Bügelteile
- einen übrigen Teil der Brillenfassung außer den Bügelteilen

umfassen, und/oder
wobei die Kopf-Metadaten auf Basis der Bügelteile berechnete Auflagebereiche für die Ohren des Kopfes umfassen.

Das Verfahren ist dadurch gekennzeichnet, dass die Segmentierung des 3D Modells der Brillenfassung in Komponenten der Brillenfassung mittels jeweiliger Schnittebenen im Raum erfolgt, welche Vertices oder Voxel des 3D-Modells unterteilen, wobei die jeweiligen Schnittebenen durch einen jeweiligen Aufpunkt p und einen jeweiligen Normalenvektor n, welcher auf der jeweiligen Schnittebene im Raum senkrecht steht, definiert werden und wobei die Segmentierung des 3D Modells der Brillenfassung basierend auf einem Vergleich

- des Skalarprodukt eines Vertex oder Voxel des 3D Modells der Brillenfassung mit dem jeweiligen Normalenvektor n mit
- einem Skalarprodukt des jeweiligen Aufpunkts p mit dem Normalenvektor n erfolgt.

Gegenüber dem aus der US 2013 088490 A1 bekannten Verfahren zur Segmentierung des 3D-Modells der Brillenfassung in Komponenten der Brillenfassung hat das Verfahren gemäß dem dritten Erfindungsaspekt den Vorteil, dass die Segmentierung rechnerisch einfach implementierbar bereitgestellt wird.

Unter Grobpositionieren wird verstanden, dass aufgrund von Informationen eine Ausgangsposition bestimmt wird. Unter Feinpositionierung wird verstanden, dass ausgehend von einer Grobpositionierung basierend auf weiteren Daten eine genauere Positionsbestimmung durchgeführt wird. Beispielsweise wäre es möglich, dass im Rahmen der Grobpositionierung eine Positionierung entlang der z-Richtung erfolgt; und anschließend im Rahmen der Feinpositionierung eine Positionierung im Wesentlichen senkrecht zur z-Richtung erfolgt.

Die Grobpositionierung kann basierend auf Informationen über den Nasenrücken erfolgen: Hierbei kann ein 3D-Punkt im Koordinatensystem der Fassung, auch als Ankerpunkt bezeichnet, mit einem Punkt auf dem Nasenrücken zur Deckung gebracht werden.

Die Feinpositionierung kann in manchen Ausführungsbeispielen das Brillengestell um weniger als 20 mm, in manchen Ausführungsbeispielen um weniger als 10 mm gegenüber der Ausgangslage, also der durch die Grobpositionierung bestimmten Position des Brillengestells, bewegen.

Die Begriffe "Grobpositionieren" und "Feinpositionieren" sind dabei relativ zueinander zu verstehen, d.h. das Feinpositionieren präzisiert die zunächst beim Grobpositionieren bestimmte Position.

Dies erfolgt dadurch, dass das Grobpositionieren einen Startpunkt bzw. eine Ausgangslage für ein darauf folgendes Feinpositionieren bestimmt, und das Feinpositionieren eine zunächst beim Grobpositionieren bestimmte Position präzisiert.

Das Grobpositionieren stellt somit ein erstes Positionieren, das Feinpositionieren ein darauf folgendes Positionieren dar.

Grobpositionieren bedeutet also, dass zunächst die Brillenfassung an eine Position positioniert wird, die eine nach dem Feinpositionieren erreichte endgültige Position annähert, z.B. in einem Bereich von 20 mm, 10 mm oder weniger, bezogen auf Maße des Kopfes. Das Feinpositionieren verändert diese Position dann zu der endgültigen Position. Das Grobpositionieren beruht dabei auf den Metadaten, während für das Feinpositionieren die Modelle selbst herangezogen werden.

Durch das Heranziehen der Kopf-Metadaten und der Fassungs-Metadaten für das Grobpositionieren kann das Grobpositionieren schnell und mit vergleichsweise geringem Rechenaufwand erfolgen. Zudem ergibt sich durch das Grobpositionieren ein Startpunkt bzw. eine Ausgangslage für das folgende Feinpositionieren, welcher das Feinpositionieren beschleunigen kann, da das Feinpositionieren bereits von einer annähernd korrekten Position der Brillenfassung gestartet wird. Dies reduziert die Gefahr, dass bei dem Feinpositionieren eine Position gefunden wird, die nicht einem gewünschten Optimum entspricht. Indem für das Feinpositionieren die 3-D Modelle des Kopfes und der Fassung selbst herangezogen werden, kann dann eine hohe Genauigkeit bei der Feinpositionierung erreicht werden, wobei wegen des vorangegangenen Grobpositionierens die Feinpositionierung dann insgesamt weniger rechenaufwändig ist.

Unter einem 3D-Modell ist dabei eine dreidimensionale Repräsentation von realen Objekten zu verstehen, die als Datensatz in einem Speichermedium, beispielsweise einem Speicher eines Computers oder einem Datenträger, vorliegen. Eine solche dreidimensionale Repräsentation kann beispielsweise ein 3D-Netz (englisch "3D mesh"), bestehend aus einem Satz von 3D-Punkten, die auch als Vertices bezeichnet werden, und Verbindungen zwischen den Punkten, die auch als Edges bezeichnet werden, umfassen. Diese Verbindung bilden im einfachsten Fall ein Dreiecksnetz (englisch triangle mesh). Bei einer derartigen Repräsentation als 3D-Netz wird nur die Oberfläche eines Objekts beschrieben, nicht das Volumen. Das Netz muss nicht notwendigerweise geschlossen sein. Wird also beispielsweise der Kopf in Form eines Netzes beschrieben, so erscheint er wie eine Maske. Näheres zu derartigen 3D-Modellen findet sich in Rau J-Y, Yeh P-C. "A Semi-Automatic Image-Based Close Range 3D Modeling Pipeline Using a Multi-Camera Configuration." Sensors (Basel, Switzerland). 2012;12(8):11271-11293. doi:10.3390/s120811271; insbesondere Seite 11289, Abbildung "Fig.16".)

Eine weitere Möglichkeit der Repräsentation eines 3D-Modells ist ein Voxel-Gitter (englisch "voxel grid"), welches eine volumenhafte Repräsentation darstellt. Dabei wird der Raum in kleine Würfel oder Quader eingeteilt, welche als Voxel bezeichnet werden. Für jedes Voxel wird im einfachsten Fall die An- oder Abwesenheit des darzustellenden Objekts in Form eines Binärwerts (1 oder 0) gespeichert. Bei einer Kantenlänge der Voxel von 1 mm und einem Volumen von 300 mm x 300 mm x 300 mm, was ein typisches Volumen für einen Kopf darstellt, erhält man somit insgesamt 27 Millionen derartiger Voxel. Derartige Voxel-Gitter sind beispielsweise in M. Nießner, M. Zollhöfer, S. Izadi, and M. Stamminger. "Real-time 3D reconstruction at scale using voxel hashing". ACM Trans. Graph. 32, 6, Article 169 (November 2013),. DOI: https://doi.org/10.1145/2508363.2508374 beschrieben.

Weitere mögliche Darstellungsformen eines 3D-Modells sind Punktwolken, beispielsweise ohne Informationen bezüglich der Verbindungen zwischen den Punkten (edges, beispielsweise Dreiecksnetz, s.o.), Punktewolke mit Normalenvektor in jedem Punkt, Darstellung in Form von Splineflächen und Teilflächen von geometrischen Grundkörpern, beispielsweise Kugel, Zylinder, Ebene. Die hier beschriebenen Verfahren können unabhängig von der gewählten Darstellungsform des 3D-Modells sein.

Unter einer Splinefläche wird eine durch eine oder mehrere Splines beschriebene Fläche verstanden, wie unter https://en.wikipedia.org/w/index.php?title=B-spline&oldid=840787047 beschrieben.

Unter geometrischen Grundkörpern werden durch ihre Oberflächen beschriebene dreidimensionale Figuren verstanden, wie sie unter https://de.wikipedia.org/w/index.php?title=K%C3%B6rper (Geometrie)&oldid=177672595 beschrieben sind.

Eine Punktwolke mit N Punkten bezeichnete eine Anzahl von N Punkten, wobei jeder der N Punkte durch drei Koordinaten beschrieben ist.

Ein Normalenvektor ist ein Vektor, der orthogonal (senkrecht) auf einer Geraden, Kurve, Ebene oder (gekrümmten) Fläche eines solchen Objekts oder einer Kombination solcher Objekte steht.

Das 3D-Modell des Kopfes und/oder das 3D-Modell der Brillenfassung kann insbesondere ein 3D-Modell mit Textur sein. Unter einem 3D-Modell mit Textur versteht man ein 3D-Modell, in dem zusätzlich die Farbinformation der Oberflächenpunkte des realen Objekts enthalten ist. Durch die Verwendung eines 3D-Modells mit Textur ist eine farbrealistische Darstellung des Kopfes und der Brillenfassung möglich.

Dabei kann die Farbinformation direkt in den Vertices als Attribut enthalten sein, zum Beispiel als RGB (Rot Grün Blau)-Farbenwert, oder es wird an jeden Vertex als Attribut ein Paar von Texturkoordinaten gehängt. Diese Koordinaten sind dann als Bildkoordinaten (Pixelpositionen) in einem zusätzlichen Texturbild zu verstehen. Die Textur beispielsweise der oben erwähnten Dreiecke des Dreiecksnetzes wird dann durch Interpolation aus den Pixeln des Texturbildes erzeugt.

Bei der Speicherung als Voxel-Gitter kann die Farbinformation in den Voxeln selbst gespeichert werden. Die Auflösung der Textur entspricht dann bei der Voxel-Darstellung der Größe der Voxel. Für hochauflösende Texturen ist die Umwandlung des Voxel-Gitters in ein Netz, welches die Oberfläche beschreibt, erforderlich, was als Oberflächenrekonstruktion (englisch surface reconstruction) bezeichnet wird. (siehe z.B. Cignoni, Paolo, et al. "Meshlab: an opensource mesh processing tool." Eurographics Italian Chapter Conference. Vol. 2008. 2008.)

Unter Metadaten sind Daten zu verstehen, welche Informationen über die Merkmale des Modells, jedoch nicht das Modell selbst enthalten. Insbesondere können die Metadaten Zusatzinformationen zu den Modellen liefern und/oder markante Punkte oder Kurven basierend auf dem jeweiligen Modell enthalten. Metadaten sind auch in dem Wikipedia-Artikel "Metadaten", Stand 5. März 2017, allgemein erläutert. Referenzen auf Wikipedia beziehen sich, sofern nichts anderes angegeben ist, auf die deutschsprachige Wikipedia (de.wikipedia.org).

Durch die Verwendung derartiger Metadaten, welche vorab erstellt werden können, ist wie oben erwähnt auf einfache Weise eine schnelle Positionierung und auch Änderung der Position der Brillenfassung auf dem Kopf möglich, verglichen mit der Verwendung der kompletten 3D-Modelle, die typischerweise wesentlich umfangreicher sind.

"Virtuell" ist das Positionieren deswegen, weil der Vorgang in einem Rechner wie einem Computer geschieht und nicht die reale Brillenfassung auf den realen Kopf gesetzt wird.

Bevorzugt umfassen die Fassung-Metadaten dabei erste Auflageinformationen, die eine oder mehrere Stellen der Brillenfassung definieren, an denen die Brillenfassung auf dem Kopf aufliegt, und/oder die Kopf-Metadaten dabei zweite Auflageinformationen, die eine oder mehrere Stellen des Kopfes definieren, an denen die Brillenfassung auf dem Kopf aufliegt. Als Stellen können dabei Punkte, Punktmengen, Kurven oder Flächen bezeichnet werden. Mittels solcher erster und/oder zweiter Auflageinformationen kann das virtuelle Anpassen der Brillenfassung beschleunigt werden, da nicht für alle Berechnungen und Schritte beim Anpassen das gesamte 3D-Modell der Brillenfassung herangezogen werden muss.

In Fällen bei denen Stellen als Flächen definiert werden, können nachfolgend und vorhergehend für Punkte, Punktmengen und Kurven beschriebene Verfahren entsprechend angewendet werden. Komponenten, beispielsweise der Bügel im Bereich der Kontaktfläche, können durch eine Abstandsfläche zu einer Raumkurve angenähert werden. Dann kann das gleiche Verfahren wie bei der Auflagekurve verwendet werden, jedoch zusätzlich einen festen Abstand zu der Raumkurve gefordert bzw. im Verfahren durch mathematische Methoden erzwungen werden.

Bevorzugt umfassen die ersten Auflageinformationen dabei einen Auflagepunkt, welcher einen Punkt im Bereich einer Nasenbrücke der Brillenfassung, insbesondere in einer Mitte der Nasenbrücke der Brillenfassung, kennzeichnet, und einen Auflagebereich, der einen Bereich der Brillenbügel der Brillenfassung kennzeichnet.

Die Begriffe Nasenbrücke und Brillenbügel werden hier wie in der DIN EN ISO 8624:2015-12, veröffentlicht im Dezember 2015 und der DIN EN ISO 7998:2006-01, veröffentlicht im Januar 2006 verwendet.

Unter einem Auflagepunkt wird ein Punkt am Kopf verstanden, der einen Kontaktpunkt der Brillenfassung mit dem Kopf definiert.

Unter einem Auflagebereich wird ein Bereich verstanden, der eine Kontaktfläche der Brillenfassung mit dem Kopf definiert. Beispielsweise kann für ein Ohr ein Ohrauflagebereich, bzw. Auflagebereiche für die Ohren des Kopfes verwendet werden.

Unter einem potentiellen Auflagebereich, beispielsweise einem potentiellen Ohrauflagebereich, wird ein Bereich verstanden, der grundsätzlich als Auflagebereich geeignet ist, beispielsweise ein Bereich einer Brillenfassung, der bei verschiedenen Kopfformen als Auflagebereich vorgesehen ist.

Auflagepunkte und Auflagebereich können als Metadaten gespeichert werden.

Mittels eines derartigen Auflagepunkts kann die Grobpositionierung der Brillenfassung an dem Kopf erfolgen. Mittels des Auflagebereichs kann die Grobpositionierung ebenfalls unterstützt werden, insbesondere auch wenn weiter unten beschriebene Formänderungen der Brillenfassung durch Biegen und/oder Positionsänderungen der Brillenbügel erforderlich sind. Nasenbrücke und Brillenbügel sind dabei Teile der Brillenfassung, an denen die Brillenfassung typischerweise auf dem Kopf aufliegt, sodass mit dieser Wahl der Metadaten einfach eine den tatsächlichen Gegebenheiten entsprechende Positionierung vornehmbar ist.

Die Fassungs-Metadaten können dabei zusammen mit dem 3D-Modell der Brillenfassung für einer Vielzahl verschiedener Brillenfassungen vorab bestimmt und in einem Speichermedium abgelegt sein, und dann für die Durchführung des Verfahrens abgerufen werden. Auf diese Weise stehen die Daten schnell zur Verfügung.

Das 3D-Modell des Kopfes kann beispielsweise mittels stereoskopischer Bildaufnahmen erstellt werden. Bei derartigen stereoskopischen Bildaufnahmen wird ein Objekt, in diesem Fall der Kopf aus mehreren Richtungen aufgenommen, wobei die Aufnahmepositionen bekannt sind. Dies kann durch mehrere starr zueinander angeordnete Kameras erfolgen. Durch Identifizierung einander entsprechender Merkmale in den aufgenommen Bildern kann dann unter Berücksichtigung der bekannten Aufnahmepositionen das Modell erstellt werden. Näheres zu einer derartigen Bestimmung von 3D-Modellen findet sich beispielsweise in H. Hirschmüller, "Stereo Processing by Semiglobal Matching and Mutual Information" in IEEE Transactions on Pattern Analysis and Machine Intelligence, vol. 30, no. 2, pp. 328-341, Feb. 2008.doi: 10.1109/TPAMI.2007.1166).

Das 3D-Modell der Brillenfassung kann beispielsweise herstellerseitig bereitgestellt werden.

Für die Bestimmung der Fassungs-Metadaten gibt es verschiedene Möglichkeiten, wobei die verschiedenen Möglichkeiten auch miteinander kombiniert werden können. Beispielsweise können verschiedene Metadaten der Fassungs-Metadaten mit unterschiedlichen Möglichkeiten bestimmt werden. Derartige Möglichkeiten werden nun erläutert.

Bei manchen Ausführungsformen werden die Fassungs-Metadaten manuell bestimmt. Eine derartige manuelle Bestimmung erlaubt eine Bestimmung der Fassung-Metadaten ohne größeren Rechenaufwand und gibt einem Benutzer eine vollständige Kontrolle über die Fassungs-Metadaten. Hierzu kann eine Benutzerschnittstelle bereitgestellt werden, bei der die Brillenfassung auf Basis des Modells graphisch auf einer Anzeige dargestellt wird. Punkte und/oder Konturen der Brillenfassung können dann manuell ausgewählt werden, beispielsweise durch Anklicken mit einer Maus und einem entsprechend dargestellten Mauszeiger oder durch direktes Markieren auf einem berührungsempfindlichen Bildschirm. Dabei kann insbesondere der oben erwähnte Auflagepunkt als einzelner Punkt angeklickt werden, und der Auflagebereich kann als Konturlinie oder Konturpolynom ausgewählt werden. Zudem können als Metadaten Drehachsen an Scharnieren der Brillenfassung manuell markiert werden.

Bei einer anderen Ausführungsform wird der Auflagebereich beispielsweise als Konturlinie an einer Unterseite jedes Brillenbügels automatisch bestimmt, beispielsweise durch einen Bilderkennungsalgorithmus. In diesem Fall ist keine Benutzereingabe erforderlich.

Schließlich wird bei anderen Ausführungsbeispielen eine vollautomatische Bestimmung der Fassungs-Metadaten bereitgestellt. Dies erfolgt bevorzugt mit Verfahren des maschinellen Lernens, wie sie im Wikipedia Artikel "Maschinelles Lernen", Stand 8. März 2017, beschrieben sind. Als Trainingsdaten für dieses maschinelle Lernen können dabei Metadaten verwendet werden, welche für eine Reihe von Brillenfassungen manuell oder teilweise manuell wie oben beschrieben erstellt wurden.

Bei einem bevorzugten Ausführungsbeispiel umfasst das Bestimmen der Fassungs-Metadaten eine Bestimmung, insbesondere automatische Bestimmung von markanten Punkten in dem 3D-Modell der Fassung. Markante Punkte sind dabei Punkte, die bestimmte, vorgegebene Eigenschaften haben, welche in sogenannten 3D-Merkmalsdeskriptoren (3D-Feature-Deskriptoren) definiert sein können. 3D-Merkmalsdeskriptoren bezeichnen Vektoren im $R^n$, welche die Eigenschaften eines markanten Punkts eines 3D-Modells beschreiben, so dass dieser Punkt auf einem ähnlichen 3D-Modell an Hand dieses Eigenschaften-Vektors wiedergefunden werden kann. Es können Punktemengen in der Umgebung des markanten Punktes verwendet werden. Beispielsweise kann eine Kugel mit einem vorgegebenen Radius, z.B. 10 mm, mit dem Punkt als Zentrum aufgespannt werden und die Eigenschaften der Punkte, die innerhalb dieser Kugel liegen untersucht werden, also von dieser Teilmenge der Punktwolke. Beispielsweise kann eine Eigenwertanalyse im Sinne der Hauptkomponentenanalyse durchgeführt werden. Hierbei können extremale Eigenwerte k1 und k2 dann als Elemente in die Meta-Daten, beispielsweise in Form eines Eigenschafts-Vektors aufgenommen werden. Unter einem Eigenschaftsvektor wird hierbei eine Repräsentationsform von Werten von Meta-Daten verstanden. Zusätzlich oder alternativ können auch die Hauptkrümmungen der Fläche in der Umgebung des Punktes bestimmt werden. Unter Hauptkrümmungen werden Informationen verstanden wie sie im Artikel https://de.wikipedia.org/w/index.php?title=Hauptkr%C3%BCmmung&oldid=172452500 beschrieben sind. Die Hauptkrümmungen können ebenfalls in den Eigenschaftenvektor aufgenommen werden. Alternativ kann ein Histogramm über alle Punkte gebildet werden und/oder Normalenvektoren der Kugel bestimmt werden. Hierzu kann die Kugel in Bereiche (Volumina) unterteilt werden und für jeden Bereich die Häufigkeit der Punkte (bzw. Normalenvektoren) ermittelt werden, die in diesem Bereich liegen. Alternativ kann die relative Häufigkeit bestimmt werden. Informationen dieser Häufigkeit, beispielsweise in Form eines Histogramms, kann ebenfalls in den Eigenschaftsvektor übernommen werden.

Ein Beispiel für solche Verfahren mittels sog. Fast Point Feature Histogramm Deskriptoren (FPFH) ist auf http://pointclouds.org/documentation/tutorials/fpfh estimation.php, abgerufen am 25.05.2018, beschrieben.

Solche Eigenschafts-Vektoren können insbesondere mit Hilfe des maschinellen Lernens bestimmt werden: Beispielsweise kann ein menschlicher Experte markante Punkte auf dem 3D Modell, beispielsweise durch Anklicken, vorgeben. Solche Bereiche können beispielsweise Punkte im Bereiche der Scharniere der Fassung eines 3D-Modells einer Brillenfassung sein, die auf allen Brillenfassungen eindeutig zu bestimmen sind; die Software extrahiert die Punktemenge innerhalb einer Kugel um die angeklickten Punkte - die Punktemenge und der vom Benutzer angegebene Typ des Punktes, auch als numerisches Label bezeichnet, werden jeweils als Trainingsdaten in ein maschinelles Lernverfahren eingegeben, z.B. in ein neuronales Netz. Das fertig trainierte Netz ist anschließend in der Lage, für jeweilige Punkte in einer gegebenen Punktwolke jeweilige Label zu bestimmen und somit markante Punkte zu identifizieren und/oder Punkte zu klassifizieren. Beispielsweise können Punkte, die nicht markant sind als nicht relevant klassifiziert werden.

Solche markanten Punkte stellen ein vergleichsweise kleines Datenvolumen dar, und zur Bestimmung der markanten Punkte können etablierte Algorithmen herangezogen werden. Näheres zu einer solchen Bestimmung markanter Punkte mittels 3D-Merkmalsdeskriptoren ist z.B. in Samuele Salti, Federico Tombari, Riccardo Spezialetti, and Luigi Di Stefano. 2015. Learning a Descriptor-Specific 3D Keypoint Detector. In Proceedings of the 2015 IEEE International Conference on Computer Vision (ICCV) (ICCV '15). IEEE Computer Society, Washington, DC, USA, 2318-2326, DOI=http://dx.doi.org/10.1109/ICCV.2015.267 beschrieben. Derartige vorgegebene Eigenschaften können z.B. eine Krümmung des Modells in der Nachbarschaft der Punkte beinhalten. Beispiele für 3D-Merkmalsdeskriptoren sind sogenannte "Fast Point Feature Histograms" (FPFH), wie sie in R.B. Ruso "Semantic 3D Object Maps for Everyday Manipulation in Human Living Environments", Dissertation an der TU München 2009, Seite 57ff. beschrieben werden.

Entsprechend können auch markante Kurven definiert werden. Markante Kurven können als 3D-Kurven definiert sein. 3D-Kurve bedeutet eine Repräsentation einer Kurve im dreidimensionalen Raum. 3D Kurven können durch eine Liste von 3D-Punkten abgelegt sein; alternativ können die 3D Kurven auch als Polynom-Kurve, in manchen Ausführungsbeispielen mit einer orthogonalen Basis, z.B. als Legendre-Polynombasis dargestellt werden. In anderen Ausführungsbeispielen können Kurven als Spline-Kurve, dargestellt mit Hilfe der B-Spline-Basisfunktionen dargestellt werden, wie in https://de.wikipedia.org/w/index.php?title=Legendre-Polynom&oldid=172427311 und https://en.wikipedia.org/w/index.php?title=B-spline&oldid=840787047 beschrieben.

Mit Hilfe solcher markanter Kurven kann beispielsweise der Auflagebereich der Bügel beschrieben werden; dabei kann ein Punkt auf der Kurve mit dem Auflagepunkt am Ohr in Übereinstimmung gebracht werden. In solchen Ausführungsbeispielen ist die Kurve Teil der Metadaten der Fassung und läuft beispielsweise an der Unterseite der Bügel entlang und beschreibt den gesamten möglichen Auflagebereich.

So bestimmte markante Punkte können dann in relevante und nicht relevante Punkte klassifiziert werden. Unter relevanten Punkten werden Punkte verstanden, welche verwendet werden um Meta-Daten zu repräsentieren. Insbesondere können relevante-Punkte ohne Redundanz oder mit möglichst geringer Redundanz die benötigten Meta-Daten repräsentieren.

Unter nicht-relevanten Punkten werden Punkte verstanden, die entweder keine Meta-Daten repräsentieren oder eine geringere Relevanz haben, beispielsweise weil die Meta-Informationen, die von dem nicht-relevanten Punkt repräsentiert werden, ebenfalls von anderen Punkten repräsentiert werden, also eine Redundanz der Informationen vorhanden ist.

Bei manuellen Verfahren kann der Benutzer die relevanten Punkte bestimmen, bei automatischen Verfahren kann die Zahl der relevanten Punkte als ein Ziel vorgeben werden; nicht-lernbasierte maschinelle Verfahren können zu jedem markanten Punkt auch ein Maß für die "Besonderheit" dieses Punktes (engl. "Saliency") bestimmen. Beispielsweise kann bei Verwendung von Hauptkrümmungen als Eigenschaft von Flächen das Produkt der beiden Hauptkrümmungen als Maß verwendet werden. Lernbasierten maschinellen Verfahren können das manuelle Auswählen imitieren, wobei die relevanten Punkte durch die Trainingsphase vorgegeben sein können.

So können nachfolgende Schritte auf die relevanten Punkte eingeschränkt werden, sodass die zu verarbeitende Datenmenge reduziert wird. Dies kann insbesondere mittels Verfahren des Überwachten Lernens (Supervised Learning) geschehen, wie sie in dem Wikipedia Artikel "Überwachtes Lernen", Stand 8. März 2017, beschrieben sind. Hierbei werden die bei der Bestimmung von markanten Punkten identifizierten Punkte beispielsweise zusammen mit einer Darstellung der Fassung auf einer Anzeige angezeigt, und den jeweiligen Punkten können dann Punkteklassen manuell zugeordnet werden. Diese Punkteklassen können dann mit dem 3D-Merkmalsdeskriptor für den jeweiligen Punkt verknüpft werden.

Diese Verknüpfung kann dann als Datensatz zu jeder Fassung hinterlegt werden, beispielsweise als .XML-Datei. Die Punkteklassen geben an, welches Element der Brillenfassung der jeweilige Punkt angibt oder wo der jeweilige Punkt auf der Brillenfassung liegt. Beispiele für Punkteklassen sind zum Beispiel "Scharnier außen oben links" für einen Punkt, der an einem linken Brillenscharnier außen und oben liegt, "Scharnier außen unten links" für einen entsprechenden Punkt, der unten liegt, entsprechende Klassen für das rechte Scharnier, "Zentrum-Brücke-Vorne" für einen Mittelpunkt der Nasenbrücke auf der Vorderseite, "Zentrum-Brücke-Hinten" für einen Punkt auf der Nasenbrücke auf einer Rückseite etc. Die Vorderseite ist dabei die Seite der Brillenfassung, welche beim Aufsetzen der Brillenfassung vom Kopf der aufsetzenden Person abgewandt ist, und die Rückseite ist die Seite der Brillenfassung, die dem Kopf der Person zugewandt ist.

Beim unterstützten Lernen gibt es verschiedene mögliche Algorithmen, um so die für die markanten Punkte erlernten Punkteklassen in ein Modell zu überführen und dieses Modell anzuwenden, um dann nach der Lernphase für einen mit einem 3D-Merkmaldeskriptor aufgefundenen Punkt die jeweilige Punkteklasse automatisch zu ermitteln. Siehe hierzu auch "Machine Learning Overview" von Open Source Computer Vision, Docs.opencv.org//3.1.0/dc/dd6/ml_intro.html, Stand 8. März 2017. Beispiele für Algorithmen sind ein Random Forest Algorithmus (siehe Breiman, Leo. "Random forests." Machine learning 45.1 (2001): 5-32.), z.B. wie implementiert durch die Klasse "cv::m!::RTrees" in der Software-Bibliothek "Open CV", welche zum Training und zur Klassifizierung verwendet werden kann, siehe "http://docs.opencv.org/3.1.0/d0/d65/classcv_1_1ml_1_1RTrees.html". Dies ist ein Beispiel für das Random Forest Verfahren. Ein anderes Random Forest Verfahren kann in der Software Matlab mittels der Klasse TreeBagger eingeführt werden.

Ein weiterer möglicher Algorithmus ist die Verwendung einer sogenannten "Support Vector Machine (SVM)", wie sie ebenfalls in der oben erwähnten Software Bibliothek Open CV bereitgestellt wird.

Mittels derartiger Algorithmen können markante Punkte dann automatisch identifiziert werden, was die Verarbeitung des 3D-Modells der Brillenfassung erleichtert, da nach der Trainingsphase dann keine Benutzereingabe mehr nötig ist, um die markanten Punkte zu identifizieren.

Bevorzugt wird nach der Identifizierung und Klassifizierung der markanten Punkte eine Koordinatentransformation durchgeführt. Durch eine derartige Koordinatentransformation können Fassungs-Metadaten und/oder 3D-Modelle der Fassung für verschiedene Fassungen auf ein gemeinsames Koordinatensystem transformiert werden. Dies erleichtert die nachfolgende Verarbeitung, da für jede Brillenfassung von dem gleichen Koordinatensystem ausgegangen werden kann.

Bei manchen Ausführungsbeispielen wird eine derartige Koordinatentransformation mittels einer Hauptkomponentenanalyse auf der Menge der als relevant klassifizierten markanten Punkte durchgeführt. Die Hauptkomponentenanalyse (im Englischen "principal component analysis", PCA) ist beispielsweise indem Wikipedia Artikel https://de.wikipedia.org/wiki/Hauptkomponentenanalyse und https://en.wikipedia.org/wiki/Principal_component_analysis beschrieben. Bei Punktemenge X von n Punkten im Raum kann beispielsweise eine Matrix X mit n Zeilen und 3 Spalten (für x, y und z Koordinaten der jeweiligen n Punkte) gebildet werden. Dann kann die 3x3 Matrix $X^{t*}X$ untersucht werden, und die Hauptkomponenten können als die größten Eigenvektoren dieser Matrix bestimmt werden.

Als Hauptachsentransformation wird eine Koordinatentransformation in das Koordinatensystem, das durch die Hauptkomponenten aufgespannt wird, verstanden.

Eine andere Möglichkeit der Koordinatentransformation ist eine regelbasierte Koordinatentransformation durch feste Zuordnung eines Koordinatenursprungs und Richtungen von Koordinatenachsen auf Basis der markanten Punkte. Unter regelbasierter Koordinatentransformation wird eine Linearkombination aus markanten Punkten zur Definition der Basisvektoren des Zielkoordinatensystems, d.h. des Koordinatensystems, in das transformiert wird, verstanden. Beispielsweise kann ein markanter Punkte der oben erwähnten Klasse "Zentrum-Brücke-Vorne" als Koordinatenursprung dienen und Differenzvektoren zwischen vorgegebenen Punkten oder Linearkombinationen aus der artigen Differenzvektoren als Koordinatenachsen. So kann die Differenz eines Punktes der oben erwähnten Klasse "Scharnier außen oben rechts" zu einem Punkt der Klasse "Scharnier außen oben links" als Koordinatenachse verwendet werden. Hierbei kann es vorteilhaft sein, wenn die markanten Punkte eindeutig bestimmt sind und/oder in einer festen Reihenfolge vorliegen. Dies kann beispielsweise Falle von manuell festgelegten Punkten, welche beispielsweise durch Auswahl durch einen Benutzer festgelegt werden, der Fall sein.

Bei einem derartigen Koordinatensystem mit einem Zentrum der Nasenbrücke als Ursprung verläuft dann eine Symmetrieebene der Brillenfassung durch diesen Ursprung. Im Folgenden wird für die Erklärungen angenommen, dass die Brillenbügel der Brillenfassung im Wesentlichen in z-Richtung orientiert sind, eine Verbindungslinie der Scharniere der Brillenfassung parallel zur x-Achse liegt und eine y-Richtung senkrecht hierzu steht. In anderen Worten ist bei aufrechter Haltung des Kopfes als x-Richtung eine horizontale Richtung entsprechend einer Verbindungslinie der Augen des Kopfes, insbesondere von Pupillenmitten der Augen des Kopfes in einer Nullblickrichtung, definiert, als y-Richtung eine vertikale Richtung definiert und als z-Richtung eine Richtung senkrecht zu der x- und y-Richtung definiert.

In Ausführungsbeispielen, bei denen die Position von Pupillenmitten der Augen des Kopfes in Nullblickrichtung nicht bekannt ist, kann die Verbindungslinie der Augen des Kopfes auch anders definiert werden, beispielsweise anhand der Augenpositionen des 3D-Modells.

Unter aufrechter Haltung des Kopfes wird eine Haltung des Kopfes verstanden, bei der die Verbindungslinie der Augen in einer Ebene parallel zur Frontalebene des Kopfes liegt und die vertikale Richtung parallel zur Schnittgerade von Frontalebene und Sagittalebene liegt. Das 3D-Modell des Kopfes kann so angeordnet werden, dass sich das 3D-Modell des Kopfes in einer aufrechten Haltung befindet.

Frontalebene und Sagittalebene sind in https://de.wikipedia.org/w/index.php?title=Frontalebene&oldid=177246560 und https://de.wikipedia.org/w/index.php?title=Transversalebene&oldid=177246519 definiert.

Es kann in manchen Beispielen möglich sein, dass das Koordinatensystem fest mit dem Kopf verbunden ist, so dass beispielsweise bei einer Kippung des Kopfes der Lagebezug zwischen dem Koordinatensystem und dem Kopf unverändert bleibt, also beispielsweise die x-Richtung immer noch entsprechend der Verbindungslinie der Augen des Kopfes verläuft. In anderen Worten kann der Kopf ortsfest im Koordinatensystem angeordnet sein.

Hierbei dient der Bezug zur aufrechten Haltung des Kopfes der Definition des Koordinatensystems von x-Richtung, y-Richtung und z-Richtung. Die z-Achse verläuft hierbei im Regelfall parallel zur Nullblickrichtung.

Die oben erwähnte Symmetrieebene ist dann die y-z-Ebene. Dieses Koordinatensystem dient jedoch nur als Beispiel, und es sind auch andere Anordnungen der Achsen oder auch eine Verwendung von Kugelkoordinaten statt kartesischer Koordinaten möglich.

Bei einem Ausführungsbeispiel werden dann vorgegebene Komponenten der Brillenfassung lokalisiert. Insbesondere kann die Position von Nasenbrücken und Scharnieren der Brillenfassung bestimmt werden. Dies kann auf Basis der klassifizierten markanten Punkte erfolgen, welche wie bereits oben erläutert manuell ausgewählt werden können oder auch automatisch wie beschrieben bestimmt werden können. Beispielsweise kann die Position der Scharniere der Brille auf Basis von markanten Punkten, die außen oder innen bei den Scharnieren bestimmt wurden, bestimmt werden, insbesondere durch Punkte von Klassen wie "Scharnier außen oben links" und "Scharnier außen unten links" wie oben beschrieben. Bei einem Ausführungsbeispiel erfolgt die Berechnung eines Scharnierachspunktes als Mittelwert derartiger markanter Punkte. Insbesondere kann ein Scharnierachspunkt links gemäß

Scharnier-Achspunkt-links = ¼ * (Scharnier-außen-oben-links + Scharnier-außen-unten-links + Scharnier-innen-oben-links + Scharnier-innen-unten-links)

berechnet werden, wobei Scharnier-Achspunkt-links die Position eines Scharnierachspunktes auf einer linken Seite der Brillenfassung bezeichnet und Scharnier-außen-oben-links, Scharnier-außen-unten-links, Scharnier-innen-oben-links und Scharnier-innen-unten-links markante Punkte entsprechender Punkteklassen bezeichnet, das heißt Punkte, die außen am linken Brillenscharnier oben links, unten links, und das jeweils innen und außen (auf einer Innenseite des Brillengestells, das heißt zur Nasenbrücke hin, und auf einer Außenseite des Brillengestells, das heißt von der Nasenbrücke weg) liegen. Für den rechten Scharnier-Achspunkt kann eine entsprechende Formel verwendet werden, indem die Punkte auf der linken Seiten durch Punkte auf der rechten Seite ersetzt werden. Auf diese Weise ist die Bestimmung eines Scharnierachspunktes, welcher auf der Scharnierachse im Inneren der Brillenfassung liegt, auf Basis von von außen sichtbaren Punkten möglich.

Die Nasenbrücke kann in ähnlicher Weise als Durchschnitt von Punkten der Klassen "Zentrum-Brücke-Vorne" und "Zentrum-Brücke-Hinten" wie oben erläutert definiert werden.

Erfindungsgemäß erfolgt zur Bestimmung der Fassungs-Metadaten eine Segmentierung des 3D-Modells der Fassung in Komponenten, wobei die Komponenten insbesondere Komponenten für die Brillenbügel und Komponenten, welche die Brillenfassung abgesehen von den Brillenbügeln beschreiben, umfassen können. Unter Segmentierung eines 3D-Modells wird eine Aufteilung der Informationen in Teilmengen verstanden, beispielsweise von 3D-Punktwolken in Teilmengen von 3D-Punktwolken.

Durch eine derartige Segmentierung können die verschiedenen Komponenten einzeln zur Bestimmung von Fassungs-Metadaten analysiert werden, was die zu verarbeitende Datenmenge reduziert. Die Segmentierung kann dabei insbesondere auf Basis der markanten Punkte und/oder auf Basis der Lokalisierung von Nasenbrücke und/oder Scharnieren wie oben erläutert erfolgen.

Hierzu werden Schnittebenen im Raum definiert, welche die Punkte (Vertices) oder Voxel des 3D-Modells unterteilen.

Die Schnittebenen können dabei durch einen jeweiligen Aufpunkt p und einen Normalenvektor n, welcher auf der Ebene senkrecht steht, definiert werden. Aufpunkt p bedeutet dabei ein Stützpunkt für eine Ebene, der die Lage der Ebene zum Koordinatensystemursprung zusammen mit dem Normalenvektor n eindeutig kennzeichnet. Der Aufpunkt kann als Vektor vorliegen. Ebenfalls können Koordinaten als Vektoren vom Koordinatensystemursprung zu den Koordinaten des Punktes repräsentiert werden. Dann gilt für einen Vertex oder Voxel des 3D-Modells, dessen Position durch Koordinaten v (entsprechend der kartesischen Koordinatendarstellung des Punktes) definiert wird, entweder $<v, n> \leq <p, n>$ oder $<v, n> > <p, n>$. Dabei bezeichnet $<,>$ das euklidische Skalarprodukt. Skalarprodukt bedeutet das euklidische Skalarprodukt, aber auch anders definierte Skalarprodukte können Anwendung finden. Der Normalenvektor n ist dabei auf eine Länge 1 normiert, das heißt $<n, n> = 1$.

Je nachdem welche der beiden Relationen gilt, liegt der Punkt v auf der einen oder der anderen Seite der Schnittebene. So ist eine einfache Segmentierung gemäß dem dritten Erfindungsaspekt, aber nicht auf diesen begrenzt, in Komponenten möglich.

Zur Segmentierung der Brillenfassung kann bei dieser Herangehensweise als Aufpunkt der Schnittebene der jeweilige Scharnierachspunkt verwendet werden, welcher wie oben beschrieben bestimmt werden kann. Als normalen Vektor für die Schnittebene kann dann die z-Achse des Koordinatensystems bei der obigen Koordinatentransformation dienen, das heißt die Richtung, in die die Brillenbügel verlaufen. Die Vertices oder Voxel, für die dann $<v, n> \leq <p, n>$ definieren den jeweiligen Brillenbügel.

Auf Basis der so aus dem 3D-Modell der Brillenfassung segmentierten Komponenten können dann weitere Metadaten berechnet werden. So kann der oben erwähnte Auflagebereich der Auflageinformationen auf Basis der aus dem 3D-Modell der Brillenfassung segmentierten Brillenbügel berechnet werden. Indem die segmentierten Komponenten verwendet werden, müssen weniger Daten bei der Bestimmung der Metadaten verwendet werden als wenn das komplette 3D-Modell verwendet werden würde, wodurch die Berechnung beschleunigt werden kann.

Zur Bestimmung des Auflagebereichs kann folgendes Verfahren für jeden der aus dem 3D-Modell der Brillenfassung segmentierten Brillenbügel verwendet werden:

Zunächst wird ein Anfangspunkt a und ein Endpunkt b des jeweiligen Brillenbügels aus dem jeweiligen segmentierten Brillenbügel des 3D-Modells der Brillenfassung bestimmt. Anfangs- und Endpunkt- des Brillenbügels bedeutet hierbei den Anfangs- und Endpunkt des Bereiches, der als Ohrauflagebereich vorgesehen ist. Bei dem oben genannten Koordinatensystem kann derjenige Punkt des segmentierten Brillenbügels, welcher einen minimalen Wert seiner z-Komponente aufweist, der Anfangspunkt a und ein Punkt, welcher einen maximalen Wert der z-Komponente aufweist, der Endpunkt b sein. Die Bestimmung kann manuell, beispielsweise durch Benutzerauswahl aus dem 3D-Modell, oder regelbasiert, beispielsweise indem ein fester Abstand zum Auflagepunkt in beide Richtungen gefordert wird, oder als ein geforderter relativer Abstand bezogen auf die Enden der Bügel, erfolgen.

Als nächstes wird ein potenzieller Ohrauflagebereich bestimmt. Z-Komponenten von Ebenen, welche diesen potenziellen Ohrauflagebereich begrenzen, können als vorgegebenes Verhältnis $\alpha$ und $\beta$ zu der Länge des Brillenbügels in z-Richtung verwendet werden, sodass gilt:

$$\alpha < (v_z - a_z) / l_z < \beta$$

$\alpha$ und $\beta$ sind dabei vorgegebene Werte, welche beispielsweise herstellerseitig für eine jeweilige Brillenfassung vorgegeben werden können oder auch als Erfahrungswerte aus vielen Brillenfassungen bestimmt sein können. $\alpha$ kann zwischen 0,3 und 0,6 liegen, z.B. $\alpha = 0,47$, und $\beta$ kann zwischen 0,6 und 0,9 liegen, z.B. $\beta = 0,76$.

$a_z$ ist die z-Komponente des Punktes a, und $l_z$ ist die Länge des Brillenbügels in z-Richtung, entsprechend $b_z - a_z$. $v_z$ ist die z-Komponente eines Vertex oder Voxels des 3D-Modells. Vertices oder Voxel, für deren z-Komponente $v_z$ die obige Beziehung gilt, gehören zum potenziellen Ohrauflagebereich, das heißt für diese Vertices oder Voxel gilt:

$$v_z > \alpha \times l_z + a_z$$

und

$$v_z < \beta \times l_z + a_z$$

Der Auflagebereich kann dann als Auflagepunktmenge oder Auflagekurve mittels einer Berechnung mit sich schrittweise verschiebendem Fenster (sliding window) ermittelt werden. Eine Auflagepunktmenge bedeutet die Menge der Punkte, die den Auflagebereich bilden bzw. eine Untermenge davon.

Eine Auflagekurve ist eine Repräsentation der Auflagepunkte als eine 3D-Kurve. Fenster bedeutet einen bestimmten dreidimensionalen Bereich, in dem Punkte betrachtet werden. Hierzu wird in z-Richtung ein Fenster von fest eingestellter Breite (beispielsweise Breite zwischen 0,5 mm und 3 mm, z.B. 1 mm in z-Richtung) und einer fest eingestellten Schrittweite (beispielsweise ebenfalls zwischen 0,5 mm und 3 mm, zum Beispiel etwa 1 mm) über den potenziellen Ohrauflagebereich geschoben. Für jede Position des Fensters wird zunächst die Menge der Vertices oder Voxel des potenziellen Auflagebereichs, die im Fenster liegen ermittelt. Anschließend wird für jeden dieser Vertices oder Voxel das Skalarprodukt s mit einem Richtungsvektor d gebildet, s = <v, d>, wobei v wieder die Koordinaten des Vertex oder Voxels angibt.

Ein Maximum $\hat{s}$ dieser Skalarprodukte s ist in der Regel eindeutig einem zugeordneten Vertex oder Voxel mit den Koordinaten $\hat{v}$ zugeordnet. Falls mehrere maximale Skalarprodukte in einem Fenster vorliegen, wird ein vorbestimmtes Vertex oder Voxel, beispielsweise das zuerst mit dem Maximalwert berechnete, als Vertex oder Voxel $\hat{v}$ für dieses Fenster genommen. Die Menge der so aufgefundenen Koordinaten $\hat{v}$ für alle Fenster, geordnet beispielsweise nach aufsteigendem z-Wert, ergibt eine Anordnung von Punkten, welche den Auflagebereich gemäß dem fünften Erfindungsaspekt, aber nicht auf diesen begrenzt, als Auflagekurve charakterisieren. Diese Kurve kann bei Ausführungsbeispielen durch Filter geglättet werden, beispielsweise mittels eines Gaußfilters, oder es kann eine Kurve an die Punkte angepasst werden, beispielsweise eine Spline-Approximation mittels B-Splines durchgeführt werden, wie diese in dem Wikipedia Artikel "Spline", Stand 9. März 2017, beschrieben ist.

Als Richtungsvektor d wird dabei ein normierter Vektor angenommen, das heißt <d, d> = 1. Dieser kann für den linken und rechten Brillenbügel jeweils fest gewählt werden. Ein Beispiel für einen fest gewählten Richtungsvektor wäre der Vektor (0, -1, 0) in dem oben definierten Koordinatensystem. In diesem Fall wäre der Auflagepunkt immer der tiefste Punkt des Brillenbügels. Präziser wird die Lage des Bügels bei nach innen gerichteter Auflagekurve, also z.B.

$$d=(\cos(\sigma), \sin(\sigma), 0) \text{ für } \sigma = 30° \text{ für den rechten Bügel}$$

und

$$d=(-\cos(\sigma), \sin(\sigma), 0) \text{ für } \sigma = 30° \text{ für den linken Bügel}$$

Bei manchen Ausführungsbeispielen enthalten die Kopf-Metadaten einen Ohrauflagepunkt, welcher beispielsweise durch manuelles Auswählen eines Punktes auf einer Darstellung der Ohren des Modells gewählt wird. In diesem Fall kann zur Bestimmung des Richtungsvektors d ein Fenster um diesen Ohrauflagepunkt in dem 3D-Modell des Kopfes gewählt werden, zum Beispiel alle Vertices oder Voxel, welche einen z-Wert aufweisen, der maximal eine vorgegebene Distanz, beispielsweise maximal zwischen 0,25 mm und 1 mm, beispielsweise maximal 0,5 mm, von diesem Ohrauflagepunkt entfernt wird. Dann wird der Mittelwert dieses aller Vertices des Fensters bestimmt, und der normierte Differenzvektor Ohrauflagepunkt minus Mittelwert als Richtungsvektor d definiert.

Auf diese Weise kann eine Auflagekurve als Auflagebereich auf relative einfache Weise automatisch bestimmt werden und dann in weiteren Schritten des Verfahrens zur Positionierung der Brillenfassung an dem Kopf verwendet werden, wie später noch näher erläutert werden wird.

Zur Beschleunigung der Anpassung können die Fassungs-Metadaten zudem erwartete Ohrauflagepunkte an den Brillenbügeln umfassen, welche manuell ausgewählt werden können. Diese erwarteten Ohrauflagepunkte können dann als Startpunkt beim virtuellen Positionieren der Brille dienen, das heißt die Brille wird zunächst mit diesen Punkten auf den Ohren platziert. Dies kann das virtuelle Positionieren beschleunigen.

Zudem können die Metadaten im Falle von Brillenfassungen, welche Nasenpads aufweisen, Punkte umfassen, welche die Position derartiger Nasenpads bezeichnen. Auch diese Punkte können manuell ausgewählt werden oder mit maschinellem Lernen bestimmt werden.

Beispielsweise kann ein vom Benutzer angeklickter Punkt als Zentrum des Nasenpads angenommen werden. Um diesen kann eine Kugel mit einem vorgegebenen Radius gezogen werden, beispielsweise mit 6 mm Radius. Dann können die sich innerhalb dieser Kugel befindlichen Punkte mittels Hauptkomponentenanalyse, wie zuvor beschrieben, analysiert werden.

Um solche Punktwolken zu analysieren kann insbesondere vor der Analyse der Kugelmittelpunkt von den Koordinaten der Punktwolke abgezogen werden, um eine um einen Ursprung zentrierte Punktemenge zu erhalten. Dies kann die nachfolgende Analyse vereinfachen.

Die Fassungs-Metadaten können zudem Biegeinformationen hinsichtlich einer Biegefähigkeit der Fassung, insbesondere der Brillenbügel umfassen. Mit derartigen Informationen kann die Brille dann beim virtuellen Positionieren virtuell durch Biegen an den Kopf angepasst werden, und diese Anpassungen können dann auf eine entsprechende reale Brillenfassung angewendet werden. Beispiele für derartige Informationen sind beispielsweise eine Information, ob und wie weit eine Rotation um Scharnierachsen möglich ist (zum Beispiel bei Kunststofffassungen mit gefedertem Scharnier) Brillenscharniere haben einen Anschlag - z.B. bei maximaler Öffnung von 180 Grad. Dieser Anschlag kann bei einigen Scharnieren mittels einer Feder realisiert sein, so dass eine Öffnung über 180 Grad hinaus möglich ist. Bei festem Anschlag ohne Feder wird bei Aufbiegung der Bügel deformiert. Es wird in der Information als z.B. ein Typ des Scharniers kodiert - also fix oder gefedert. Zusätzlich wird die Flexibilität des Bügels kodiert; bei gefederten Scharnieren wird dabei ein starres Modell des Bügels verwendet. Ein weiteres Beispiel ist eine Information über eine mögliche Verstellung der Inklination am Brillenbügel, das heißt eine Rotation der Brillenbügel um die x-Achse relativ zur restlichen Brillenfassung. Dies ist bei vielen Metallfassungen durch Verbiegung der Bügel an den Scharnieren nach oben oder unten möglich.

Diese Information kann auch angeben, dass keine Verstellung der Inklination möglich ist. Die Information kann beispielsweise als Ja/Nein-Information (Verstellen der Inklination möglich oder nicht) oder auch als Winkelbereich, innerhalb dem die Inklination verändert werden kann, vorliegen.

Zudem kann ein Aufbiegen der Brillenbügel selbst definiert werden, beispielsweise mittels einer Biegefunktion. Als eine derartige Biegefunktion kann beispielsweise ein multivariates Polynom über der z-Achse und einem Offset in x- und xy-Richtung beschreiben. Zudem kann auch die durch ein Aufbiegen der Bügel hervorgerufene Deformation des Fassungsrandes der Brillenfassung modelliert werden, beispielsweise durch ein multivariates Polynom oder eine multivariate Spline-Funktion (zum Beispiel Tensorprodukt einer B-Spline-Basis, siehe De Boor, Carl, et al. "A practical guide to splines". Vol. 27. New York: Springer-Verlag, 1978, Kapitel XVII.).

Ein derartiges Aufbiegen ist insbesondere bei dünnen Brillenbügeln oder sehr elastischen Brillenbügeln möglich.

Die obigen Informationen können für Verbiegungen und Anpassungen der Brillenbügel in verschiedenen Richtungen, beispielsweise in x- und y-Richtung gemäß dem obigen Koordinatensystem, bereitgestellt werden.

Als nächstes werden Varianten und Bestimmungsmöglichkeiten für die Kopf-Metadaten gemäß verschiedenen Ausführungsformen diskutiert.

Bevorzugt umfassen die Kopf-Metadaten eine Position der Augen, welche dann für eine horizontale Ausrichtung der Brillenfassung verwendet werden können. Diese Metadaten geben beispielsweise die Position der Augen, beispielsweise der Pupillen, insbesondere der Pupillenmitten, in Nullblickrichtung (siehe DIN EN ISO 13666:2013-10, 5.33), als Punktkoordinaten an. Des Weiteren können die Kopf-Metadaten Ohraufsetzpunkte, wie bereits oben erwähnt, umfassen, welche einen Auflagepunkt der Brillenbügel auf den Ohren angeben. Auch andere Aufsetzpunkte sind möglich, die einen Auflagepunkt des Brillengestells am Kopf angeben, beispielsweise ein Aufsetzpunkt an der Nase des Kopfes. Durch diese Metadaten ist eine einfache Positionierung der Brillenbügel auf den Ohren möglich.

Zudem können die Kopf-Metadaten einen Aufsetzpunkt und/oder eine Beschreibung des Nasenrückens, beispielsweise in Form einer 3D-Kurve, welche einen Bereich des Nasenrückens, auch als Nasenrückenbereich bezeichnet, beschreibt, umfassen. Diese Metadaten können zur Grobpositionierung der Brillenfassung am Kopf dienen. Der Aufsetzpunkt ist dabei ein Punkt an oder bei der Nase im 3D-Modell des Kopfes, welcher insbesondere mit einer Grobpositionierung mit dem oben erwähnten Auflagepunkt der Fassungs-Metadaten in Übereinstimmung gebracht werden kann.

Derartige Kopf-Metadaten können wie bereits für die Fassung-Metadaten wie oben diskutiert manuell bestimmt werden, beispielsweise durch Auswählen entsprechender Punkte oder Kurven in einer Darstellung des Kopfes auf Basis des 3D-Modells. Sie können aber auch ganz oder teilweise automatisch bestimmt werden, beispielsweise mit maschinellem Lernen wie oben diskutiert.

Bei anderen Ausführungsformen werden zusätzlich zu dem 3D-Modell des Kopfes zweidimensionale Bilddaten des Kopfes zur automatischen Bestimmung von Kopf-Metadaten herangezogen. Derartige 2D-Bilder stehen ohnehin zur Verfügung, wenn das 3D-Modell mittels Kameraaufnahmen des Kopfes von mehreren kalibrierten Positionen erstellt wird. Kalibrierte Positionen bedeutet, dass die die Positionen der Bildaufnahmen bekannt sind, sodass auf Basis der aufgenommen Bilder und der Positionen dann das 3D-Modell berechnet werden kann. Alternativ können 2D-Bilder auch aus dem 3D-Modell mit Textur durch Bildsynthese (auch als Rendern bezeichnet) erzeugt werden. Informationen hierzu sind beispielsweise im Artikel "Bildsynthese" auf Wikipedia, Stand 10. März 2017, zu finden.

In derartigen 2D-Bildern können dann Gesichtsmerkmale der Person wie Augenlider, Augenbrauen, Nasenrücken, Augen und dergleichen durch Bilderkennungsverfahren (auch als Objektdetektoren bezeichnet) identifiziert werden. Geeignete Verfahren hierzu sind beispielsweise in der Software-Bibliothek "dlib", "http://blog.dlib.net/2014/08/real-time-face-pose-estimation.html", Stand 10. März 2017, bereitgestellt oder in V. Kazemi et al., "One Millisecond Face Alignment with an Ensemble of Regression Trees", X. Zhu et al., "Face detection, pose estimation and landmark localization in the wild", CVPR 2012, auch dargelegt auf der Homepage des Erstautors "https://www.ics.uci.edu/~xzhu/face/", Stand 10. März 2017, beschrieben.

Diese so identifizierten Punkte oder Bereiche in dem zweidimensionalen Bild werden anschließen auf das 3D-Modell (beispielsweise das 3D-Netz) des Kopfes projiziert und stehen somit als 3D-Punke als Kopf-Metadaten zur Verfügung. Bei einer bevorzugten Ausführungsform wird zur Projektion eine inverse Projektionsmatrix verwendet. Die Projektionsmatrix setzt sich dabei aus einer Festkörpertransformation von dem Koordinatensystem des Modells, beispielsweise 3D-Netzes, in das Kamerakoordinatensystem der verwendeten Kamera und der Kameramatrix zusammen. Diese Matrizen sind, wenn wie oben beschrieben, eine Kameraeinrichtung mit kalibrierten Positionen für die Bildaufnahme zur Aufnahme des 3D-Modells sowie des 2D-Bildes verwendet wird, gegeben oder werden im Rahmen der Bestimmung des 3D-Modells aus den aufgenommen Bildern bestimmt.(Siehe Richard Hartley and Andrew Zisserman. 2000. Multiple View Geometry in Computer Vision. Cambridge University Press, New York, NY, USA.)

Auf diese Weise kann die Projektion schnell erfolgen, da die erforderlichen Matrizen bereits aus der Bestimmung des 3D-Modells bekannt sind. Bei einer alternativen Ausführungsform können Gesichtsmerkmale in den 2D-Bildern auch von Hand markiert werden, wozu das 2D-Bild auf einer Anzeige dargestellt werden kann. Auch Mischformen sind möglich, bei welchen die Gesichtsmerkmale automatisch wie oben erläutert bestimmt werden und dann von Hand noch eine Korrektur erfolgen kann.

Der oben erwähnte Nasenrückenbereich kann als eine 3D-Kurve bereitgestellt werden. Hierzu kann ein Verfahren entsprechend dem Verfahren zum Bestimmen des Auflagebereichs der Brillenbügel wie bereits oben beschrieben zum Einsatz kommen. Insbesondere kann das oben beschriebene Verfahren mit gleitendem Fenster verwendet werden, wobei als Richtungsvektor d der obigen Formeln eine Richtung des Kopfes (welche beispielsweise eine Koordinatenachse wie die z-Achse des 3D-Modells) sein kann, welche in Kopfrichtung nach hinten orientiert ist. Diese Richtung kann bei der Bestimmung des Kopfes mitbestimmt werden. Der Richtungsvektor d entspricht hier der Richtung der Kopfdrehung bzw. der Blickrichtung wie in der europäischen Patentanmeldung 17153559.4 beschrieben, die mittels eines Zentriergeräts bestimmt werden kann, welches in der europäischen Patentanmeldung 17153556.0 offenbart ist; die Verschieberichtung des Fensters ist hier die y-Achse des 3D-Modells des Kopfes - also die vertikale Achse; das gesamte Modell des Kopfes wird verwendet oder ein Ausschnitt mittels y-Koordinate des Mittelwerts aus der Pupillenposition und y-Koordinate der Nasenspitze - bestimmt als v_max für den das Skalarprodukt $s = <v, d>$ (wie oben) maximal wird

Wie für den Auflagebereich kann auch hier eine Filterung beispielsweise mittels Gaußfiltern und/oder eine Spline-Approximation durchgeführt werden.

Als Parametrisierung der Kurve des Nasenrückens wird hier das Intervall auf der y-Achse verwendet. Man erhält also eine Funktion, die für jeden y-Wert den zugehörigen 3D-Punkt auf dem Nasenrücken liefert.

Der oben erwähnte Aufsatzpunkt kann dann, insbesondere gemäß dem fünften Erfindungsaspekt, aber nicht auf diesen Erfindungsaspekt begrenzt, auf Basis dieser 3D-Kurve für den Nasenrückenbereich bestimmt werden. Hierzu wird bei einer Ausführungsform eine Ebene, in welcher eine Verbindungslinie der Augenpositionen des 3D-Modells liegt und in welcher die Blickrichtung liegt, mit der 3D-Kurve, auch als Nasenrückenkurve bezeichnet, geschnitten, was einen Startpunkt s für die Bestimmung des Auflagepunkts dient. In einer Umgebung dieses Punktes s, beispielsweise innerhalb eines vorgegebenen Bereichs (einer vorgegebenen Distanz, beispielsweise $\pm$ 3 mm, $\pm$ 2 mm etc.) wird dann derjenige Punkt der Nasenrückenkurve bestimmt, welcher den kleinsten Wert in Blickrichtung aufweist, das heißt am weitesten hinten (Richtung des Hinterkopfs des 3D-Modells) gelegen ist. Zum y-Wert dieses Punktes kann noch ein fester Offset in y-Richtung addiert werden, um den y-Wert und mittels obiger Parametrisierung der Kurve den Aufsetzpunkt zu ermitteln. Auf diese Weise kann ein Aufsetzpunkt ermittelt werden, welcher zusammen mit dem Auflagepunkt der Fassungs-metadaten für eine anfängliche Positionierung der Brillenfassung verwendet werden kann, die relative nahe an einer endgültigen Position liegt, was eine nachfolgende Optimierung beschleunigen kann.

Die Kopf-Metadaten können dann zusammen mit dem 3D-Modell des Kopfes abgespeichert werden und somit für verschiedene Fassung verwendet werden. Dies erspart Neuberechnungen der Kopf-Metadaten für verschiedene Fassungen.

Das virtuelle Positionieren auf Basis der Metadaten kann bevorzugt eine Grobpositionierung auf Basis der Metadaten umfassen, um einen Startpunkt für die nachfolgende weitere Anpassung bereitzustellen. Hier kann insbesondere der oben erwähnte Aufsetzpunkt auf dem Nasenrücken mit dem Auflagepunkt der Brillenfassung in Übereinstimmung gebracht werden. Dabei kann die z-Achse der Fassung (das heißt die Richtung der Brillenbügel) mit einer entsprechenden Achse des 3D-Modells des Kopfes, welche die Blickrichtung bezeichnet, zur Deckung gebracht werden.

Nach diesem Teil der Grobpositionierung kann bei einer Ausführungsform die Fassung derart um die x-Achse rotiert, das heißt die Vorneigung der Fassung verändert, dass der Auflagebereich der Brillenbügel auf den Ohrauflagepunkten anliegt. Dies wird zunächst für jeden Bügel getrennt durchgeführt. Hierzu wird bei einer Ausführungsform ein Schnitt des Auflagebereichs mit einer Zylinderfläche berechnet, wobei die Zylinderachse mit der Rotationsachse der Fassung (in dem obigen Koordinatensystem der x-Achse) übereinstimmt. Die Rotationsachse verläuft dabei durch den Auflagepunkt. Der Radius des Zylinders ist der Abstand des jeweiligen Ohrauflagepunktes von der Rotationsachse. Der Schnittpunkt auf dem Zylinder ergibt direkt den Winkel, um den die Fassung vorgeneigt wird oder zurückgeneigt wird. Der Zylinderschnitt kann dabei durch iterative Linienzylinderschnitte berechnet werden. Dabei kann der Auflagebereich (beispielsweise die oben ermittelte Auflagekurve) zunächst in vergröberter Segmentdarstellung verwendet werden, das heißt mit geringerer Auflösung, um die Berechnung zu beschleunigen. Beispielsweise kann im einfachsten Fall als vergröberte Segmentdarstellung die Verbindungslinie zwischen dem oben erwähnten Anfangspunkt a und Endpunkt b verwendet werden.

Diese Bestimmung mittels iterativer Linienzylinderschnitte kann wie folgt vorgenommen werden: Der Schnittpunkt dieser Verbindungslinie mit dem Zylinder sei mit S bezeichnet. Dann wird bei einer iterativen Verfeinerung die z-Koordinate zs von S verwendet, um einen zugehörigen realen Kurvenpunkt zu ermitteln. Dieser liegt zwar nicht mehr auf der Zylinderfläche, kann dennoch als Näherung verwendet werden, um den Winkel zu bestimmen. Zusätzlich kann bei nun ein weiterer Iterationsschritt durchgeführt werden. Dabei werden Punkte $k(zs - \varepsilon)$ und $k(zs + \varepsilon)$ auf der Kurve mit einem Abstand $\varepsilon$ um $z_s$ verwendet, die in einer nächsten Iteration als neue Anfangs- und Endpunkte anstatt von a und b fungieren.

Nach dieser Positionierung erfolgt ein Biegen der Fassung, wozu die oben erwähnten Biegeinformationen der Fassungs-Metadaten, welche eine Biegefähigkeit der Fassung beschreiben, herangezogen werden.

Hierzu kann wieder das oben beschriebene Verfahren mit Zylinderschnitten angewendet werden. Falls die Biegeinformation wie oben diskutiert als multivariate Biegefunktion vorliegt, kann eine Optimierung durchgeführt werden, bei der als Kostenfunktion ein Abstand des Ohrauflagepunktes von dem Auflagebereich des Bügels verwendet wird. Eine Kostenfunktion ist dabei allgemein eine Funktion, welche sich in Abhängigkeit von einem oder mehreren zu optimierenden Parametern verändert und zur Optimierung auf ein Maximum oder Minimum gebracht wird. Der für die Stärke der Biegung verantwortliche Parameter der Biegefunktion wird dabei als zu optimierender Parameter gewählt. Bei einer einfachen polynomialen Biegefunktion für die Aufbiegung in x-Richtung ist eine Näherung der mathematischen Umkehrfunktion ebenfalls durch ein Polynom möglich. Hierfür wird für eine Verbiegung in x-Richtung an der Stelle z die Biegefunktion $D_z(x)$ additiv als Polynom modelliert; die Umkehrfunktion $D_z^{-1}(d)$ davon wird ebenfalls durch ein Polynom angenähert, z.B. indem man an einzelnen diskreten Stellen [x1,x2,..,xn] die Funktion abtastet und eine neue Polynomapproximation auf [$D_z$ (x1), $D_z$ (x2),..., $D_z$ (xn)] mit Werten [$D_z^{-1}(D_z$ (x1)), $D_z^{-1}(D_z$ (z2)) ,..., $D_z^{-1}(D_z$ (xn))] durchführt. In diesem Fall kann direkt die Aufbiegung der Auflagekurve auf den x-Wert des Ohransatzpunktes ermittelt werden, indem die Umkehrfunktion für z=Auflageposition und x=Differenz Ohransatz zu Auflagepunkt angewendet wird.

Das Biegen der Fassung kann auch eine Anpassung der Inklination an den Bügel durch Rotation in z-Richtung durchgeführt werden (das heißt eine Verbiegung der Bügel um die x-Achse). Auch hier kann wieder die oben erwähnte Zylinderschnittmethode verwendet werden, wobei der Ohransatzpunkt für die Konstruktion des Zylinders verwendet wird und mit der Auflagekurve geschnitten wird.

Bei einer Ausführungsform gemäß dem ersten Erfindungsaspekt, aber nicht nur auf diesen Erfindungsaspekt begrenzt, ist die Feinpositionierung der Fassung zumindest im Wesentlichen auf eine x-y-Ebene beschränkt. Unter x-y-Ebene wird hierbei eine Ebene verstanden, die parallel zur x-Richtung als zur y-Richtung ausgedehnt ist, welche also von zwei Vektoren, wovon ein Vektor in x-Richtung und ein Vektor in y-Richtung verläuft, aufgespannt wird und somit senkrecht zur z-Richtung steht. Ein Vektor in z-Richtung ist dann ein Normalenvektor der x-y Ebene. Eine Verschiebung in der x-y-Ebene kann auch als Verschiebung in x-y-Richtung bezeichnet werden. Insbesondere kann die x-y-Ebene den Ankerpunkt der Brillenfassung nach der Grobpositionierung umfassen.

Das Beschränken der Feinpositionierung zumindest im Wesentlichen auf die x-y Ebene bedeutet dabei, dass eine Positionsveränderung in z-Richtung zumindest nicht mehr als 10% oder nicht mehr als 5% einer Positionsveränderung in der x-y-Ebene beträgt, insbesondere auch 0 sein kann. Dabei wird die Fassung in x-y-Richtung verschoben, bis keine Kollision zwischen dem 3D-Modell der Brillenfassung und dem 3D-Modell des Kopfes vorliegt.

Eine Positionsveränderung der Brillenfassung kann mittels Verschieben der Brillenfassung herbeigeführt werden.

Unter Verschieben der Brillenfassung versteht man eine Translationsbewegung der Brillenfassung, welche durch einen Verschiebungsvektor charakterisiert werden kann. Der Verschiebungsvektor kann die Verschiebung zwischen der durch die Grobpositionierung erhaltenen Ausgangslage der Brillenfassung und der Position der Brillenfassung am Ende des Feinpositionierungsschritts beschreiben.

Die Länge des Verschiebungsvektors kann als Distanz des Verschiebens bezeichnet werden. Hierbei kann bei manchen Ausführungsformen ein Verschieben der Brillenfassung um eine erste Distanz in der x-y-Ebene erfolgen und ein Verschieben der Brillenfassung um eine zweite Distanz entlang der z-Richtung erfolgen. Wie zuvor beschrieben, kann das Verschieben zumindest im Wesentlichen auf die x-y-Ebene beschränkt sein. Dies kann beispielsweise bedeuten, dass in manchen Ausführungsbeispielen die zweite Distanz nicht größer als 10% der ersten Distanz ist. In manchen Ausführungsbeispielen ist die zweite Distanz nicht größer als 5% der ersten Distanz. In manchen Ausführungsbeispielen ist die zweite Distanz 0% der ersten Distanz.

Für diese Feinpositionierung kann die Oberfläche des 3D-Modells des Kopfes geringfügig abgesenkt werden (beispielsweise zwischen 0,3 mm und 1 mm), um eine Verformung der Haut an Auflageflächen der Brillenfassung zu berücksichtigen. Auf diese Weise kann eine realistischere Anpassung ermöglicht werden. Keine Kollision bedeutet in diesem Fall, dass das 3D-Model der Fassung und das 3D-Modell des Kopfes keine gemeinsamen Raumbereiche aufweisen. Nach der Feinpositionierung kann der oben genannte Biegevorgang erneut durchgeführt werden, um die Anpassung weiter zu verbessern.

Bei einer bevorzugten Ausführungsform kann nach diesem Schritt eine prinzipielle Eignung der Brillenfassung für den jeweiligen Kopf ermittelt werden, indem überprüft wird, ob ein unterer Rand der Brillenfassung mit einem Wangenbereich des Kopfes in den jeweiligen Modellen kollidiert.

Bei Fassungen mit Nasenpads, wie sie häufig bei Metallfassungen vorliegt, kann das virtuelle Positionieren weiter ein Positionieren der Nasenpads umfassen. Diese Anpassung wird bei einem Ausführungsbeispiel als Rotation der Nasenpads um ein Rotationszentrum modelliert. Hierzu können die Nasenpads vom restlichen Teil der Brillenfassung segmentiert werden, was wie oben erwähnt geschehen kann. Diese Anpassung kann nachdem oben genannten Biegen vorgeführt werden. Die Feinpositionierung kann bei jeder Änderung der Nasenpads erneut durchgeführt werden.

Als Rotationsachsen können Hauptachsen der Nasenpads gewählt werden, welche beispielsweise mit der oben erwähnten Hauptkomponentenanalyse bestimmt werden können. Die Rotation kann auf zwei Winkel um die beiden sich aus der Hauptkomponentenanalyse ergebenden Hauptachsen zu den größten Eigenwerten eingeschränkt werden Als Kostenfunktion für eine Optimierung der Position der Nasenpads kann ein Kehrwert der Fläche, auf der die Nasenpads die Nase berühren, verwendet werden, um eine möglichst breite Auflage zu erreichen. Auf diese Weise kann auch die Position der Nasenpads virtuell angepasst werden, was dann auf eine reelle Fassung übertragen werden kann. Auf diese Weise ist die Anpassung der Nasenpads an der realen Fassung an den realen Kopf nicht oder nur noch in sehr geringem Umfang nötig.

Bei manchen Ausführungsformen kann bei dieser Veränderung der Inklination eine bestimmte voreinstellbare Vorneigung (beispielsweise 9°) vorgegeben werden. Diese fest eingestellte Vorneigung bleibt dann durch Veränderung der Inklination erhalten, auch wenn die Brille später verschoben wird, wie später näher erläutert werden wird.

Das Anzeigen des Kopfes mit der darauf positionierten Brillenfassung kann mittels herkömmlicher Bildsyntheseverfahren geschehen, siehe den oben erwähnten Wikipedia-Artikel zur Bildsynthese. Hierzu können als Textur insbesondere Kamerabilder verwendet werden, welche auch zum Erstellen des 3D-Modells dienten, da dies einen natürlichen Bildeindruck ergibt. Für die Bildsynthese kann eine Beleuchtung eingestellt oder vorgegeben werden, und die Bildsynthese kann dann beispielsweise mittels sogenanntem Raytracing (Strahlverfolgung) erfolgen, siehe Wikipedia-Artikel "Ray Tracing", Stand 10. März 2017.

Hierfür können virtuelle Lichtquellen entsprechend einer Umgebungsbeleuchtung beim Aufnehmen der Bilder des Kopfes verwendet werden. Bevorzugt wird diese Umgebungsbeleuchtung bei dem Aufnehmen der Bilder bestimmt. Um diese Umgebungsbeleuchtung zu bestimmen, kann insbesondere an dem Kamerasystem, welches zur Bildaufnahme für die Erstellung des 3D-Modells des Kopfes verwendet wird, eine Panoramakamera (das heißt eine Panoramakamera mit großem Bildwinkel) verwendet werden, welche die Umgebung aufnimmt, um Lichtquellen zu identifizieren. Bei anderen Ausführungsformen können derartige Systeme zur Bildaufnahme zum Erstellen des 3D-Modells eine Beleuchtungseinrichtung in bekannter Position aufweisen, die dann auch als Position einer Beleuchtung für die Bildsynthese verwendet wird. Bei dieser Bildsynthese können Brillengläser in der Fassung mit berücksichtigt werden, um dem Betrachter einen möglichst natürlichen Bildeindruck zu geben. In diesem Fall können insbesondere Reflexe, welche sich bei Verwendung von Spiegelungsschichten ergeben, berücksichtigt werden. Bei einer bevorzugten Ausführungsform sind Modelle für verschiedene Typen von Entspiegelungsschichten vorhanden, so wie Modelle für verschiedene Glastypen, um dem Benutzer einen Eindruck über verschiedene Schichten zu geben. Bei der Bildsynthese erscheint die Wirkung von Entspiegelungsschichten dabei insbesondere in Form von Reflexen in der Seitenansicht.

Das Verfahren kann weiterhin ein Verändern der Position der Brillenfassung auf Basis von Benutzereingaben umfassen. So kann der Benutzer den Sitz einer Brille nach seinen Wünschen anpassen, oder ein Optiker kann noch Modifikationen vornehmen. Hierzu kann dem Benutzer eine geeignete Benutzeroberfläche bereitgestellt sein, beispielsweise kann die Navigation durch Mausbewegungen oder durch Berühren eines berührungsempfindlichen Bildschirms (Touchpad) erfolgen.

Das Verfahren kann mit anderen Verfahren zur Brillenanpassung, beispielsweise Zentrierverfahren, kombiniert werden, wie sie beispielsweise in der europäischen Patentanmeldung Nr. 17153560.2 beschrieben sind. Dabei kann insbesondere eine virtuelle Zentrierung nach den dort beschriebenen Verfahren nach der obigen Anpassung der Fassung vorgenommen werden, was die Genauigkeit der Zentrierung verbessert.

Wählt der Benutzer bei dieser Interaktion einen Aufsetzpunkt der Brillenfassung auf der Nase, welcher von dem Aufsetzpunkt der Metadaten abweicht, kann der Aufsetzpunkt der Metadaten auf dem vom Benutzer gewählten Aufsetzpunkt verändert werden. Beim virtuellen Anpassen weiterer Fassungen kann dann dieser geänderte Aufsetzpunkt, welcher von dem Benutzer bevorzugt wird, verwendet werden.

Bei der Interaktion kann der Benutzer das Modell drehen und von der Seite betrachten. Dabei kann die Drehung begrenzt werden, sodass beispielsweise Bereiche, für die keine Textur vorliegt, wie ein Hinterkopf des Benutzers, nicht betrachtbar sind. Hierzu kann beispielsweise bei einem Bewegen Richtung Hinterkopf eine virtuelle Beobachtungsposition an das 3D-Modell des Kopfes heranrücken, und/oder die Sensitivität der Bewegung gegenüber Eingabe des Benutzers (Mauszeiger, Touchpad und dergleichen) kann beim Drehen in Richtung Hinterkopf nachlassen. Zudem kann der Benutzer auch die Fassung um die x-y-Achse drehen, um Asymmetrien im Gesicht zu berücksichtigen.

Das oben genannte Verfahren kann mittels einer Vorrichtung zur virtuellen Brillenanpassung durchgeführt werden, welche einen Prozessor und eine Anzeige aufweist, wobei auf dem Prozessor ein entsprechendes Computerprogramm mit einem Programmcode zur Durchführung des Verfahrens läuft. Das Computerprogramm kann auf einem Speicher der Vorrichtung abgespeichert sein oder auch über eine Cloud bereit gestellt sein. Dabei ist zu beachten, dass die Recheneinrichtung auch mittels eines verteilten Systems implementiert sein kann, welches verschiedene separate Komponenten aufweist. Beispielsweise kann die Berechnung der Metadaten oder die Berechnung des Modells des Kopfes aus aufgenommen Bilddaten auf einem vergleichsweise leistungsstarken Rechner, beispielsweise einem externen Server, welcher auch Coprozessoren wie Graphikprozessoren aufweisen kann, durchgeführt werden. Durch die Verwendung der Metadaten ist die Positionierung der Brille auf dem Kopf und das nachfolgende Darstellen weniger rechenintensiv, sodass dies auch auf leistungsschwächeren Einheiten, wie beispielsweise auf mobilen Endgeräten wie Tablets oder Smartphones, in entsprechenden Anwendungsprogrammen oder auch über das Internet mittels eines Browsers durchführbar ist.

Die Erfindung wird nachfolgend anhand von bevorzugten Ausführungsbeispielen unter Bezugnahme auf die beigefügte Zeichnungen näher erläutert. Es zeigen:

Fig. 1A eine Vorrichtung zur virtuellen Brillenanpassung gemäß einem Ausführungsbeispiel,

Fig. 1B ein Beispiel für eine Implementierung einer Kameraeinrichtung der Fig. 1A,

Fig. 2 ein Flussdiagram eines Verfahrens gemäß einem Ausführungsbeispiel,

Figuren 3A bis 3D Ansichten zur Veranschaulichung von 3D-Modellen eines Kopfes,

Fig. 4 eine Darstellung zur Veranschaulichung eines 3D-Modells einer Brillenfassung,

Fig. 5 ein Flussdiagram eines Verfahrens zur Bestimmung von Fassung-Metadaten gemäß einem Ausführungsbeispiel,

Figuren 6A und 6B Darstellung zur Veranschaulichung des manuellen Bestimmens von Metadaten,

Figuren 7A und 7B Darstellungen von Brillenfassungen,

Fig. 8 ein Flussdiagram eines Verfahrens zum Bestimmen einer Auflagekurve,

Fig. 9 ein Flussdiagram eines Verfahrens zur Brillenanpassung,

Figuren 10A und 10B Darstellungen zur Veranschaulichung von Schritten des Verfahrens der Fig. 9, und

Fig. 11 eine Darstellung zur Veranschaulichung einer Rotation der Brillenfassung.

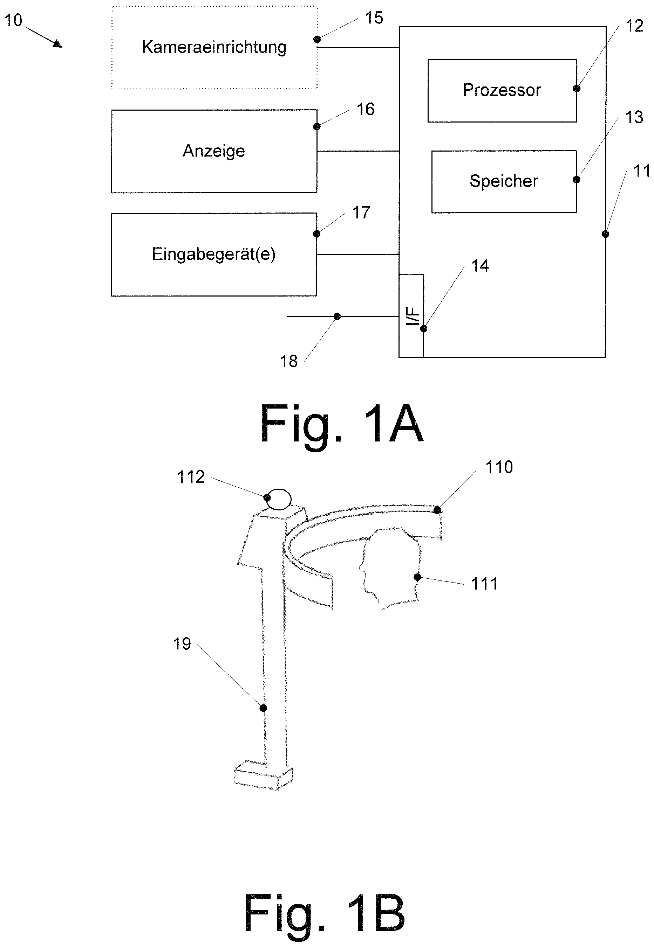

Die Fig. 1A zeigt ein Ausführungsbeispiel einer Vorrichtung zur virtuellen Brillenanpassung gemäß einem Ausführungsbeispiel. Die Vorrichtung der Fig. 1A umfasst eine Recheneinrichtung 11, einen Prozessor 12 sowie einen Speicher 13. Der Speicher 13 dient zum Abspeichern von Daten und umfasst bei dem Ausführungsbeispiel von Fig. 1A einen Speicher mit wahlfreiem Zugriff (Random Access Memory, RAM), einen Nur-Lesespeicher (Read Only Memory, ROM) sowie ein oder mehrere Massenspeichermedien (Hard Disc, Solid State Disc, optisches Laufwerk etc.). In dem Speicher 13 ist ein Programm abgelegt, mittels dem wenn es auf dem Prozessor 12 ausgeführt wird, eine virtuelle Brillenanpassung wie oben beschrieben durchgeführt wird.

Die Vorrichtung der Fig. 1A verfügt weiter über eine Anzeige 16, auf welcher ein Kopf einer Person zusammen mit einer Brillenfassung angezeigt wird, wenn das Computerprogramm auf dem Prozessor 12 ausgeführt wird. Über ein oder mehrere Eingabegeräte 17, beispielsweise Tastatur und Maus, können Benutzereingaben vorgenommen werden. Zusätzlich oder alternativ kann die Anzeige 16 ein berührungsempfindlicher Bildschirm (Touchscreen) sein, um Eingabe vorzunehmen.

Die Vorrichtung der Fig. 1A umfasst weiterhin eine Schnittstelle 14 zu einem Netzwerk 18, über das Daten empfangen werden können. Insbesondere können hier 3D-Modelle von Brillenfassungen mit Textur empfangen werden.

Bei manchen Ausführungsbeispielen werden über die Schnittstelle 14 auch Daten zu einer weiteren Recheneinrichtung gesendet, um dort Berechnungen, wie die angesprochenen Berechnungen von Metadaten auszuführen.

Zum Erstellen eines 3D-Modells eines Kopfes einer Person, an welche die Brille anzupassen ist, umfasst die Vorrichtung der Fig. 1A optional eine Kameraeinrichtung 15, über welche mehrere Bilder der Person aus verschiedenen Richtungen aufgenommen werden können und hieraus wie beschrieben das 3D-Modell bestimmt werden kann.

Die Fig. 1B zeigt eine Ausführungsform für eine Kameraeinrichtung 15 der Fig. 1A. Bei dem Ausführungsbeispiel der Fig. 1B ist eine halbkreisförmige Anordnung 110 von Kameras an einer Säule 19 befestigt. Eine Person kann sich dann derart hinstellen, dass ein Kopf 111 der Person wie in Fig. 1B gezeigt in der halbkreisförmigen Anordnung 110 positioniert ist und aus verschiedenen Richtungen aufgenommen werden kann. Hieraus kann dann wie bereits erläutert ein 3D-Modell des Kopfes 111 erstellt werden, wobei sich aus den Bildaufnahmen auch die Textur des Modells ergibt. Zudem weist die Kameraeinrichtung der Fig. 1B eine Panoramakamera 112 auf, um eine Beleuchtung bei der Bildaufnahme ermitteln zu können, was dann wie beschrieben für eine Bildsynthese verwendet werden kann.

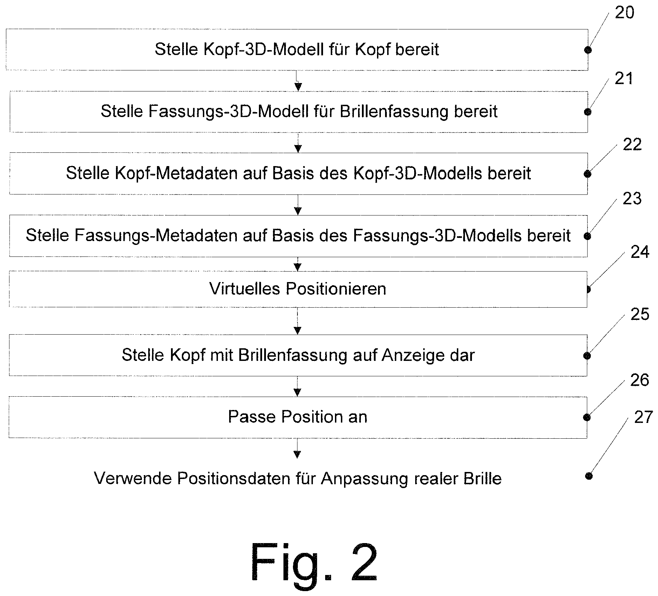

Die Fig. 2 zeigt ein Verfahren gemäß einem Ausführungsbeispiel. In Schritt 20 der Fig. 2 wird ein 3D-Modell eines Kopfes eines Benutzers bereitgestellt, beispielsweise auf Basis von Kameraaufnahmen wie oben beschrieben, insbesondere mit Aufnahmen der Kameraeinrichtung 15 der Fig. 1A. In Schritt 21 wird ein Fassungs-3D-Modell für eine anzupassende Brillenfassung bereitgestellt, beispielsweise durch einen Hersteller.

In Schritt 22 werden Kopf-Metadaten auf Basis des Kopf-3D-Modells bereitgestellt, wie bereits oben beschrieben, und in Schritt 23 werden Fassungs-Metadaten auf Basis des Fassungs-3D-Modells bereitgestellt, wie ebenso oben beschrieben. Die Schritte 20 bis 23 können auch in anderer Reihenfolge ausgeführt werden. So kann Schritt 22 mit Schritt 21 vertauscht werden. Das Bereitstellen der Metadaten in Schritt 22 und 23 kann durch Neuberechnung der Metadaten oder durch Lesen zuvor berechneter Metadaten aus einem Speicher erfolgen.

Die Metadaten können dabei wie oben diskutiert berechnet und bereitgestellt werden.

In Schritt 24 wird dann die Brillenfassung virtuell positioniert, d.h. an den Kopf unter Verwendung der Kopf-Metadaten und der Fassungsmetadaten angepasst. Hierfür kommen die bereits diskutierten Verfahren zum Einsatz. Das virtuelle Positionieren umfasst also ein Grobpositionieren auf Basis der Kopf-Metadaten und Fassungs-Metadaten und ein Feinpositionieren auf Basis der 3D-Modelle des Kopfes und der Brillenfassung.

In Schritt 25 wird der Kopf mit der Brillenfassung dann auf einer Anzeige dargestellt, im Falle der Vorrichtung der Fig. 1A auf der Anzeige 16. So kann der Benutzer dann das Aussehen der Brillenfassung auf dem Kopf beurteilen.

In Schritt 26 kann dann bei Bedarf die Position der Brillenfassung auf dem Kopf wie beschrieben verschoben werden, beispielsweise auf der Nase verschoben werden. Dies kann durch den Benutzer oder auch durch einen Optiker geschehen.

Optional können dann Anpassungsdaten, welche die Anpassung der Brille an den Kopf beschrieben werden, dann zur Anpassung einer realen Brille verwendet werden. Beispielsweise können die Bügel der realen Brillenfassung wie bei der virtuellen Anpassung des Modells gebogen werden.

Das Verfahren der Fig. 2 kann für mehrere Brillenfassungen wiederholt werden, um dem Benutzer einen Eindruck des Aussehens verschiedener Brillenfassungen zu geben. Auf Basis der Darstellungen kann sich der Benutzer dann für eine Brillenfassung entscheiden.

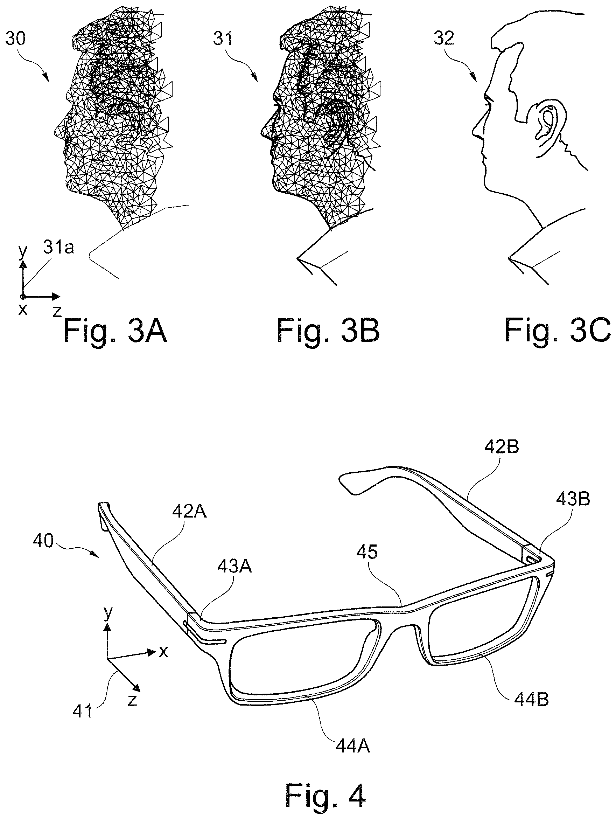

Verschiedene Schritte des Verfahrens der Fig. 2 werden nun unter Bezugnahme auf die Figuren 3-10 näher erläutert.

Die Figuren 3A und 3C zeigen eine Darstellung zur Erläuterung eines 3D-Modells eines Kopfes. Mit 31a ist ein Beispiel für ein verwendbares Koordinatensystem bezeichnet, wobei das Koordinatensystem fest - wie obenstehend erläutert - mit dem Kopf verbunden ist, also beispielsweise bei einer Drehung des Kopfes seine Lage relativ zum Kopf nicht ändert. In Fig. 3A ist dabei ein 3D-Modell 30 in Form eines Dreiecknetzes mit einer Vielzahl von Knoten, die durch Linien verbunden sind, dargestellt. In fig. 3B ist eine Kombination 31 aus dem Dreiecksnetz und einer Textur dargestellt. Die Fig. 3C zeigt eine Darstellung 32, wie sie auf Basis des Modells auf einem Bildschirm erfolgen kann, bei welchem nur die Textur sichtbar ist, nicht jedoch die einzelnen Vertices, die in den Figuren 3A und 3B zur Verdeutlichung explizit dargestellt sind. Die Fig. 4 zeigt eine Darstellung einer Brillenfassung auf Basis des Modells zusammen mit einem Koordinatensystem 41. Die Brillenfassung 40 der Fig. 4 weist einen rechten Brillenbügel 42A, einen linken Brillenbügel 42B, ein rechtes Scharnier 43A, ein linkes Scharnier 43B, einen rechten Fassungsrand 44A, einen linken Fassungsrand 44B sowie eine Nasenbrücke 45 auf.

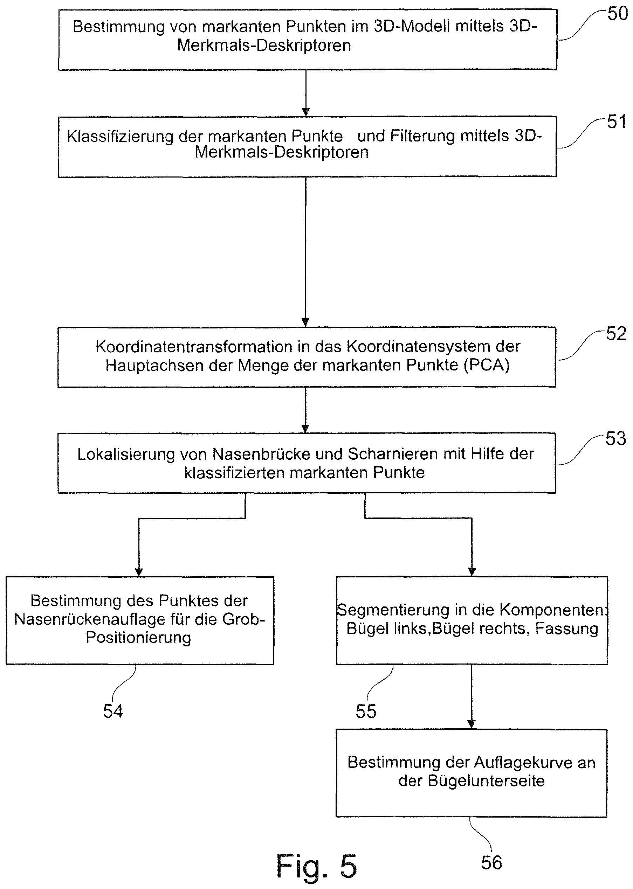

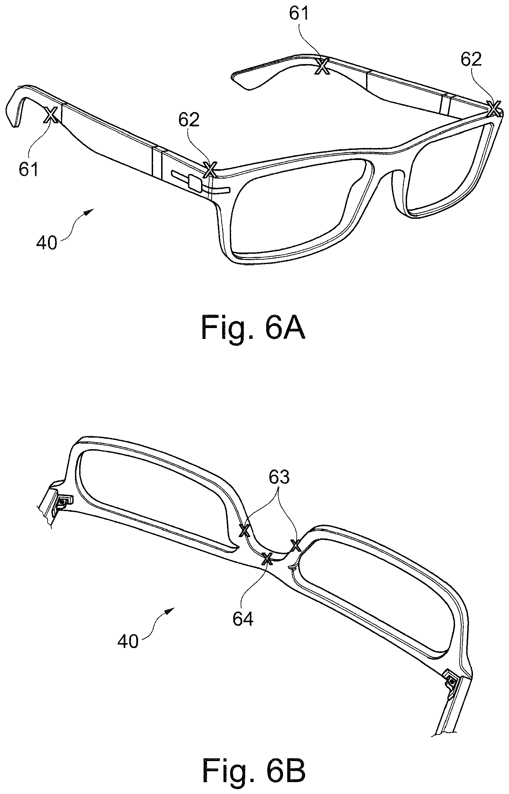

Die Fig. 5 zeigt ein Flussdiagramm eines Verfahrens zum Bestimmen von Fassungsmetadaten, das heißt ein Beispiel für eine Implementierung des Schritts 23 der Fig. 2. In Schritt 50 werden markante Punkte im 3D-Modell der Fassung mittels 3D-Merkmalsdeskriptoren bestimmt. In Schritt 51 werden diese markanten Punkte klassifiziert und gefiltert. Alternativ zur Bestimmung mittels 3D-Merkmalsdeskriptoren können Punkte auch manuell markiert werden, wie dies in Fig. 6A und 6B veranschaulicht ist. Hierzu wird die Brillenfassung 40, welche bereits unter Bezugnahme auf die Fig. 4 beschrieben wurden, auf einer Anzeige dargestellt, und relevante Punkte werden markiert. Die Figuren 6A und 6B zeigen hierzu als Beispiele Ohrauflagepunkte 61, Scharnierpunkte 62, Positionen von Nasenpads 63 sowie einen Mittelpunkt 64 der Nasenbrücke, welcher als Auflagepunkt dienen kann. Es ist zu bemerken, dass der Auflagepunkt

EP 3 657 236 B1

nicht direkt auf der Brille liegen muss, sondern insbesondere im Falle von Nasenpads auch von der tatsächlichen Fassung beabstandet sein kann.

In Schritt 52 der Fig. 5 erfolgt eine Koordinatentransformation in das Koordinatensystem der Hauptachse der in Schritt 51 klassifizierten markanten Punkte durch eine Hauptkomponentenanalyse (PCA, Principle Component Analysis), und in Schritt 53 werden Nasenbrücke und Scharniere mit Hilfe der klassifizierten markanten Punkte lokalisiert. Techniken hierfür wurden ebenfalls bereits oben beschrieben.

In Schritt 54 wird dann als Auflagepunkt ein Punkt der Nasenrückenauflage für die Grobpositionierung bestimmt, wie bereits beschrieben. In Schritt 55 wird das 3D-Modell der Brillenfassung in Komponenten segmentiert (linker Brillenbügel, rechter Brillenbügel und die übrige Fassung), und in Schritt 56 wird ein Auflagebereich an der Bügelunterseite in Form einer Auflagekurve bestimmt, wie bereits beschrieben.

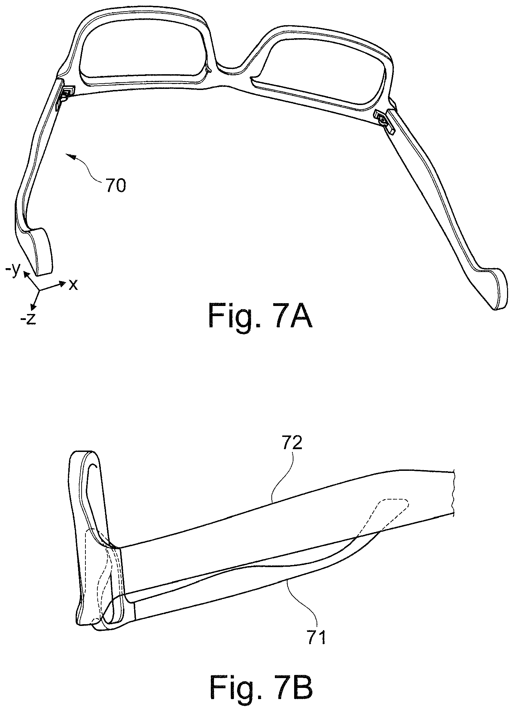

Die Bügelunterseite, auf der die Auflagekurve bestimmt wird, ist in Fig. 7A für eine Brillenfassung 70 und in einer entsprechenden vergrößerten Ansicht der Fig. 7B für die Brillenbügel 71 und 72 als Einzelpunktdarstellung dargestellt. Durch Verwendung des Auflagebereichs müssen Berechnungen nicht mit Hilfe einer derartigen Vielzahl von Punkten erfolgen, sondern es kann die Auflagekurve verwendet werden.

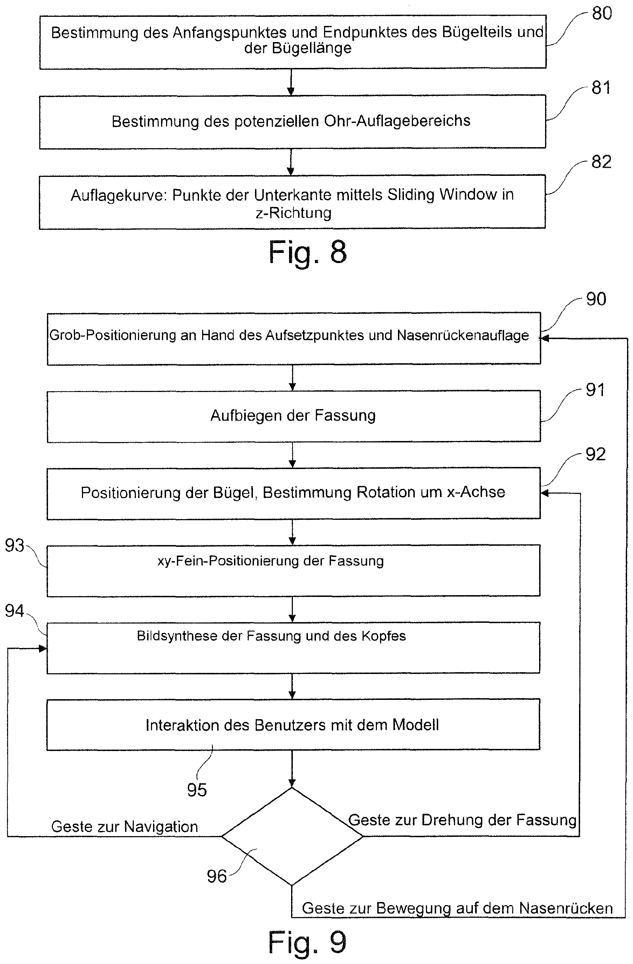

Die Fig. 8 zeigt ein Flussdiagram eines Verfahrens zum Bestimmen der Auflagekurve, das heißt eine detailliertere Implementierung des Schritts 56 der Fig. 5. In Schritt 80 werden Anfangspunkt und Endpunkt des jeweiligen Bügelteils (linker Brillenbügel oder rechter Brillenbügel) beschrieben. Das Verfahren der Fig. 8 wird dabei für den linken Brillenbügel und den rechten Brillenbügel getrennt durchgeführt. In Schritt 81 wird ein potenzieller Ohrauflagebereich bestimmt, und in Schritt 82 wird die Auflagekurve mittels der beschriebenen "Sliding Window"-Technik bestimmt. Die Schritte 80-82 werden dabei wie bereits oben detaillierter beschrieben durchgeführt.

Die Fig. 9 zeigt ein Flussdiagram eines Verfahrens zum virtuellen Anpassen und Positionieren einer Brillenfassung auf einem Kopf, wobei die oben erläuterten Modelle und Metadaten verwendet werden. Die Fig. 9 stellt ein Implementierungsbeispiel für die Schritte 24-26 der Fig. 2 dar.

In Schritt 90 der Fig. 9 erfolgt eine Grobpositionierung, wobei der Auflagepunkt der Metadaten der Brillenfassung und der Aufsetzpunkt der Metadaten des Kopfes in Übereinstimmung gebracht werden. Dann erfolgt in Schritt 91 ein Aufbiegen der Fassung, wobei hier die bereits erläuterten Zylinderschnittverfahren zum Einsatz kommen. Bei Fassungen, bei welchen eine Inklination verändert werden kann, das heißt die Inklination durch Rotation des Bügels um die x-Achse verändert werden kann (es wird das Koordinatensystem 41 der Fig. 4 verwendet), erfolgt dies in Schritt 92. Brillenfassungen, bei welchen die Fassungs-Metadaten anzeigen, dass eine derartige Rotation nicht möglich ist, kann der Schritt 92 übersprungen werden.

In Schritt 93 wird dann eine Feinpositionierung der Fassung in der x-y-Ebene senkrecht zur z-Richtung vorgenommen, wobei hier die bereits oben für die Feinpositionierung beschriebenen Techniken zum Einsatz kommen. In Schritt 94 erfolgt dann eine Bildsynthese der Fassung und des Kopfes entsprechend der Positionierung in den Schritten 90-93, wobei wie erläutert Lichtquellen berücksichtigt werden können. In Schritt 95 erfolgt dann eine Interaktion des Benutzers mit dem dargestellten Modell, das heißt der Anzeige der Fassung und des Kopfes, mit einem oder mehrerer Eingabegeräten (zum Beispiel den Eingabegeräten 17 der Fig. 1A). Hierdurch kann beispielsweise eine wie in Schritt 96 angedeutete Navigation erfolgen, das heißt das angezeigte Modell kann gedreht, vergrößert oder verkleinert werden. Auf Basis dieser Eingaben erfolgt dann eine erneute Durchführung des Schrittes 94, das heißt das Bild wird entsprechend der Navigation neu angezeigt. Es kann auch eine Eingabe, auch als Geste bezeichnet, zur Drehung der Fassung erfolgen, unter anderem um wie beschrieben Asymmetrien im Gesicht auszugleichen. In diesem Fall erfolgt dann die Berechnung der Positionierung ab Schritt 92 erneut. Schließlich kann die Brille auf dem Nasenrücken auf und ab bewegt werden. Dies entspricht einer Veränderung der anfänglichen Grobpositionierung, und so wird das Verfahren in diesem Fall ab Schritten 90 erneut durchgeführt, wobei die neu festgelegte Position auf dem Nasenrücken als Grobpositionierung des Schrittes 90 verwendet wird.

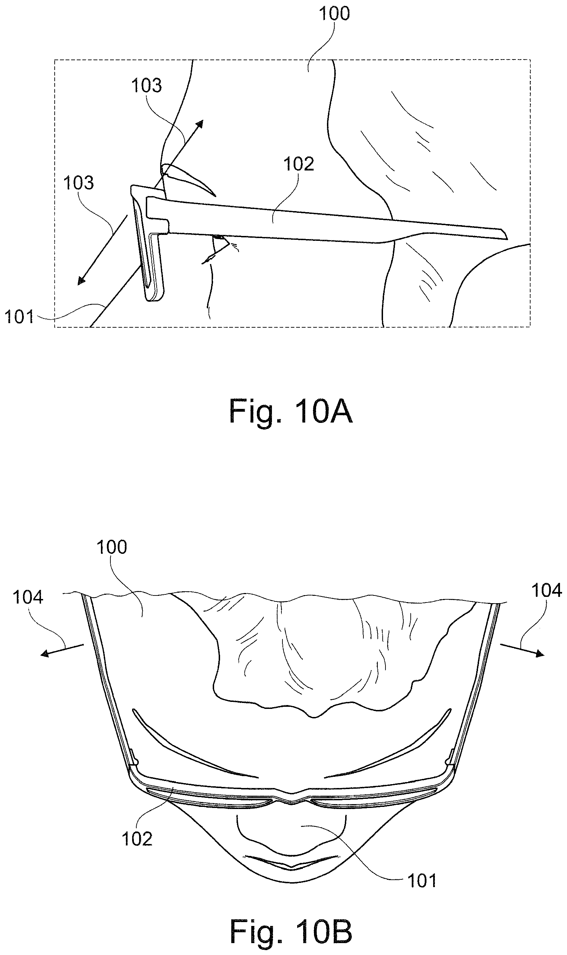

Die Figuren 10A und 10B zeigen Darstellungen eines Kopfes 100 zusammen mit einer Brillenfassung 102 zur Veranschaulichung von Verfahrensschritten der Fig. 9. Die Fig. 10A veranschaulicht wie die Brillenfassung 102 auf einem Nasenrücken 101 des Kopfes 100 entsprechend Pfeilen 103 nach oben und unten verschoben werden kann. Dies ist ein Beispiel für die Bewegung auf dem Nasenrücken gemäß Schritt 96. Die Fig. 10B veranschaulicht ein Aufbiegen der Bügel der Brillenfassung 102 entsprechend Pfeilen 104, wie sie in Schritt 91 der Fig. 9 durchgeführt wird.

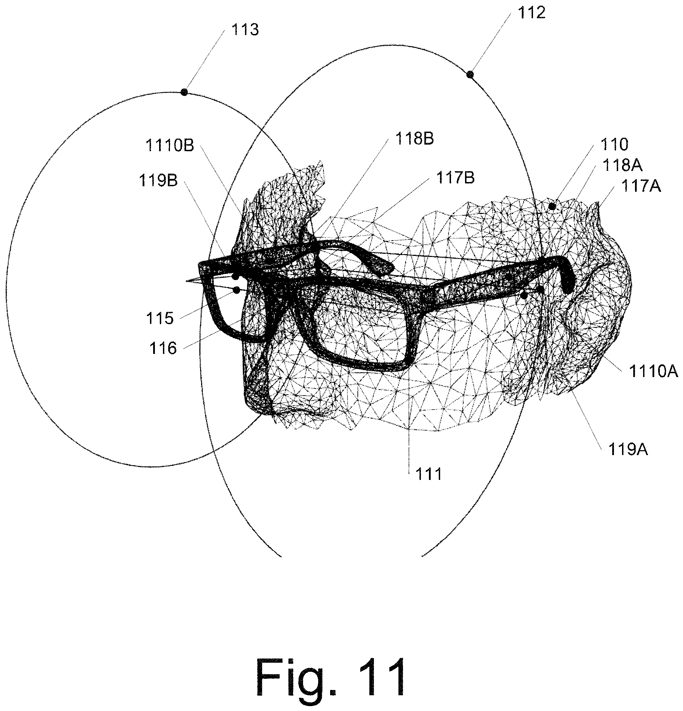

Die Fig. 11 zeigt eine Darstellung, um die oben bereits erläuterte Rotation der Fassung um die x-Achse mittels eines Zylinderschnitts erläutert. Die Fig. 11 zeigt ein 3D-Modell eines Kopfes 110 und ein 3D-Modell einer Brillenfassung 111 jeweils als 3D-Netz. Bei der Grobpositionierung wurde die Fassung zunächst anhand eines Auflagepunktes 116 positioniert. Eine Achse 115 verläuft in x-Richtung durch den Auflagepunkt 116. Ein Zylinder, angedeutet durch Kreise 112, 113 weist einen Radius zu einem Ohrauflagepunkt des 3D-Modells des Kopfes 110 auf. Der Schnittpunkt des Zylinders mit einem Auflagebereich der Brillenfassung, die durch das 3D-Modell der Brillenfassung 111 dargestellt wird, mit dem Zylinder ergibt eine Richtung für die Brillenbügel der Brillenfassung und somit einen Winkel, um die die Brillenfassung um die Achse 115 zu rotieren ist. Im vorliegenden Fall wird ausgehend von einer Position, in der die Brillenbügel in Richtung von Linien 119A, 119B verlaufen, eine Rotation durchgeführt, sodass die Brillenbügel nun in Richtung der

18

Linien 1110A, 1110B verlaufen.

Mit den beschriebenen Verfahren und Vorrichtungen kann somit virtuell eine genaue Anpassung einer Brillenfassung an einem Kopf vorgenommen werden, welche dann optional gemäß Schritt 27 der Fig. 2 auch zur Anpassung einer realen Brillenfassung verwendet werden kann.

**Patentansprüche**

1. Computerimplementiertes Verfahren zur virtuellen Brillenanpassung, umfassend:

   virtuelles Feinpositionieren einer Brillenfassung auf Basis eines 3D-Modells (30, 31) eines Kopfes und eines 3D-Modells (40) der Brillenfassung,
   virtuelles Grobpositionieren der Brillenfassung auf dem Kopf auf Basis von Kopf-Metadaten für das 3D-Modell (30,31) des Kopfes und von Fassungs-Metadaten für das 3D-Modell der Brillenfassung vor dem virtuellen Feinpositionieren,
   wobei das Bereitstellen der Fassungs-Metadaten eine Segmentierung des 3D-Modells der Brillenfassung in Komponenten der Brillenfassung umfasst, wobei die Komponenten bevorzugt mindestens eine Komponente aus der Gruppe

   - Bügelteile
   - einen übrigen Teil der Brillenfassung außer den Bügelteilen

   umfassen, und/oder
   wobei die Kopf-Metadaten auf Basis der Bügelteile berechnete Auflagebereiche für die Ohren des Kopfes umfassen,
   **dadurch gekennzeichnet, dass** die Segmentierung des 3D Modells der Brillenfassung in Komponenten der Brillenfassung mittels jeweiliger Schnittebenen im Raum erfolgt, welche Vertices oder Voxel des 3D-Modells unterteilen, wobei die jeweiligen Schnittebenen durch einen jeweiligen Aufpunkt p und einen jeweiligen Normalenvektor n, welcher auf der jeweiligen Schnittebene im Raum senkrecht steht, definiert werden und wobei die Segmentierung des 3D Modells der Brillenfassung basierend auf einem Vergleich

   - des Skalarprodukt eines Vertex oder Voxel des 3D Modells der Brillenfassung mit dem jeweiligen Normalenvektor n
   mit
   - einem Skalarprodukt des jeweiligen Aufpunkts p mit dem Normalenvektor n erfolgt.

2. Computerimplementiertes Verfahren nach Anspruch 1, wobei v den Vertex oder Voxel des 3D Modells der Brillenfassung bezeichnet, $<v, n>$ das Skalarprodukt des Vertex oder Voxel v des 3D Modells der Brillenfassung mit dem jeweiligen Normalenvektor n bezeichnet und $<p, n>$ das Skalarprodukt des jeweiligen Aufpunkts p mit dem Normalenvektor n bezeichnet und wobei gilt, dass:

   falls $<v, n> \leq <p, n>$ erfüllt ist, der Vertex oder Voxel v auf eine erste Seite der Schnittebene verortet wird und falls $<v, n> > <p, n>$ erfüllt ist, der Vertex oder Voxel v auf einer zweiten Seite der Schnittebene verortet wird.

3. Verfahren nach Anspruch 1 oder 2, wobei mindestens ein Aufpunkt p einer der jeweiligen Schnittebenen im Raum ein Scharnierachspunkt ist.

4. Verfahren nach einem der Ansprüche 1-3, **dadurch gekennzeichnet, dass** das Verfahren zum Bereitstellen der Fassungs-Metadaten ein Identifizieren von markanten Punkten in dem 3D-Modell der Brillenfassung und/oder ein Klassifizieren von markanten Punkten in dem 3D-Modell der Brillenfassung umfasst,
   wobei die markanten Punkte Punkte sind, die bestimmte, vorgegebene Eigenschaften haben, welche in sogenannten 3D-Merkmalsdeskriptoren definiert sind, und
   wobei relevante Punkte Punkte sind, welche verwendet werden, um Metadaten zu repräsentieren, und
   wobei nicht relevante Punkte Punkte sind, die entweder keine Metadaten repräsentieren oder redundant bei der Repräsentation von Metadaten sind.

5. Verfahren nach Anspruch 4, **dadurch gekennzeichnet, dass** das Verfahren weiter eine Anwendung einer Koordinatentransformation auf Basis der identifizierten markanten Punkte auf wenigstens eines aus der Gruppe

- das 3D-Modell der Brillenfassung
- die identifizierten markanten Punkte
- die Fassungs-Metadaten

umfasst.

6. Computerprogramm mit einem Programmcode, der, wenn er auf einem Prozessor ausgeführt wird, das Verfahren nach einem der Ansprüche 1-5 ausführt.

7. Vorrichtung zur virtuellen Brillenanpassung, umfassend:

einen Prozessor, und
eine Anzeige,
**gekennzeichnet durch**
ein Computerprogramm nach Anspruch 6 zur Ausführung auf dem Prozessor.

**Claims**

1. Computer-implemented method for virtual fitting of a pair of spectacles, comprising:

fine virtual positioning of a spectacle frame on the basis of a 3D model (30, 31) of a head and a 3D model (40) of the spectacle frame,
approximate virtual positioning of the spectacle frame on the head on the basis of head metadata for the 3D model (30, 31) of the head and frame metadata for the 3D model of the spectacle frame before the fine virtual positioning,
wherein the provision of the frame metadata comprises a segmentation of the 3D model of the spectacle frame into components of the spectacle frame, wherein the components preferably comprise at least one component of the group

- earpiece parts
- a remaining part of the spectacle frame apart from earpiece parts

and/or
wherein the head metadata comprise resting regions for the ears of the head, which regions are calculated on the basis of the earpiece parts,
**characterized in that** the segmentation of the 3D model of the spectacle frame into components of the spectacle frame is implemented by means of respective sectional planes in space, which subdivide vertices or voxels of the 3D model,
wherein the respective sectional planes are defined by a respective reference point p and a respective normal vector n, which is perpendicular to the respective sectional plane in space, and wherein the segmentation of the 3D model of the spectacle frame is implemented on the basis of a comparison of

- the scalar product of a vertex or voxel of the 3D model of the spectacle frame with the respective normal vector n
with
- a scalar product of the respective reference point p with the normal vector n.

2. Computer-implemented method according to Claim 1, wherein v denotes the vertex or voxel of the 3D model of the spectacle frame, $\langle v, n \rangle$ denotes the scalar product of the vertex or voxel v of the 3D model of the spectacle frame with the respective normal vector n and $\langle p, n \rangle$ denotes the scalar product of the respective reference point p with the normal vector n, and wherein the following applies:

the vertex or voxel v is located on a first side of the sectional plane if $\langle v, n \rangle \leq \langle p, n \rangle$ is satisfied and
the vertex or voxel v is located on a second side of the sectional plane if $\langle v, n \rangle > \langle p, n \rangle$ is satisfied.

3. Method according to Claim 1 or 2, wherein at least one reference point p of one of the respective sectional planes in space is a hinge axis point.

**4.** Method according to any one of Claims 1-3, **characterized in that** the method for providing the frame metadata comprises identifying prominent points in the 3D model of the spectacle frame and/or classifying prominent points in the 3D model of the spectacle frame,
wherein the prominent points are points that have certain, predetermined properties, which are defined in so-called 3D feature descriptors, and wherein relevant points are points that are used to represent metadata, and
wherein non-relevant points are points that either represent no metadata or are redundant when representing metadata.

**5.** Method according to Claim 4, **characterized in that** the method further comprises an application of a coordinate transformation on the basis of the identified prominent points to at least one of the group of:

- the 3D model of the spectacle frame
- the identified prominent points
- the frame metadata.

**6.** Computer program comprising a program code which, when executed on a processor, carries out the method according to any one of Claims 1-5.

**7.** Apparatus for virtual fitting of a pair of spectacles, comprising:

a processor and
a display,
**characterized by**
a computer program according to Claim 6 to be executed on the processor.


## Revendications

**1.** Procédé mis en œuvre par ordinateur pour adapter virtuellement des lunettes, comprenant :

le positionnement fin virtuel d'une monture de lunettes sur la base d'un modèle 3D (30, 31) d'une tête et d'un modèle 3D (40) de la monture de lunettes,
le positionnement grossier virtuel de la monture de lunettes sur la tête sur la base de métadonnées de tête pour le modèle 3D (30, 31) de la tête et de métadonnées de monture destinées au modèle 3D de la monture de lunettes avant le positionnement fin virtuel,
dans lequel la fourniture des métadonnées de monture comprend une segmentation du modèle 3D de la monture de lunettes en composantes de la monture de lunettes, dans lequel les composantes comprennent de préférence au moins une composante du groupe qui comporte

- des parties formant branches,
- une autre partie de la monture de lunettes que les parties formant branches, et/ou

dans lequel les métadonnées de tête comprennent des zones d'appui sur les oreilles de la tête, qui sont calculées sur la base des parties formant branches,
**caractérisé en ce que** la segmentation du modèle 3D de la monture de lunettes en composantes de la monture de lunettes est effectuée au moyen de plans de coupe respectifs dans l'espace qui subdivisent des sommets ou des voxels du modèle 3D, dans lequel les plans de coupe respectifs sont définis par un point d'appui p respectif et un vecteur normal n respectif, qui est perpendiculaire au plan de coupe respectif dans l'espace, et dans lequel la segmentation du modèle 3D de la monture de lunettes est effectuée sur la base d'une comparaison entre

- le produit scalaire d'un vertex ou d'un voxel du modèle 3D de la monture de lunettes avec le vecteur normal respectif n
et
- un produit scalaire du point d'appui p respectif avec le vecteur normal n.

**2.** Procédé mis en œuvre par ordinateur selon la revendication 1, dans lequel v désigne le sommet ou le voxel du modèle 3D de la monture de lunettes, <v, n> désigne le produit scalaire du sommet ou du voxel v du modèle 3D de la monture de lunettes avec le vecteur normal n respectif et <p, n> désigne le produit scalaire du point d'appui p respectif avec le vecteur normal n et étant entendu que :

si <v, n> ≤ <p, n> est satisfait, le sommet ou le voxel v est situé sur un premier côté du plan de coupe et
si <v, n> > <p, n> est satisfait, le sommet ou le voxel v est situé sur un second côté du plan de coupe.

3. Procédé selon la revendication 1 ou 2, dans lequel au moins un point d'appui p de l'un des plans de coupe respectifs dans l'espace est un point d'axe charnière.

4. Procédé selon l'une des revendications 1 à 3, **caractérisé en ce que** le procédé de fourniture de métadonnées de monture comprend une identification de points saillants dans le modèle 3D de la monture de lunettes et/ou une classification de points saillants dans le modèle 3D de la monture de lunettes,
dans lequel les points saillants sont des points présentant certaines propriétés prédéterminées qui sont définies dans des descripteurs dits de caractéristiques 3D, et
dans lequel les points pertinents sont des points qui sont utilisés pour représenter des métadonnées, et
dans lequel les points non pertinents sont des points qui soit ne représentent pas de métadonnées, soit sont redondants lors de la représentation de métadonnées.

5. Procédé selon la revendication 4, **caractérisé en ce que** le procédé comprend en outre l'application d'une transformation de coordonnées sur la base des points saillants identifiés à au moins l'un des éléments du groupe qui comporte

- le modèle 3D de la monture de lunettes
- les points saillants identifiés
- les métadonnées de monture.

6. Programme informatique comprenant un code de programme qui, lorsqu'il est exécuté sur un processeur, met en œuvre le procédé selon l'une des revendications 1 à 5.

7. Dispositif d'adaptation virtuelle de lunettes, comprenant :

un processeur, et
un afficheur,
**caractérisé par**
un programme informatique selon la revendication 6 destiné à être exécuté sur le processeur.

10

Kameraeinrichtung — 15

Anzeige — 16

Eingabegerät(e) — 17

Prozessor — 12

Speicher — 13

11

I/F — 14

18

# Fig. 1A

112

110

111

19

# Fig. 1B

| Stelle Kopf-3D-Modell für Kopf bereit | 20 |

| Stelle Fassungs-3D-Modell für Brillenfassung bereit | 21 |

| Stelle Kopf-Metadaten auf Basis des Kopf-3D-Modells bereit | 22 |

| Stelle Fassungs-Metadaten auf Basis des Fassungs-3D-Modells bereit | 23 |

| Virtuelles Positionieren | 24 |

| Stelle Kopf mit Brillenfassung auf Anzeige dar | 25 |

| Passe Position an | 26 |

Verwende Positionsdaten für Anpassung realer Brille — 27

# Fig. 2

30

31

32

31a

y

x    z

Fig. 3A          Fig. 3B          Fig. 3C

42B

42A

43B

40

43A

45

y

x

z

41

44B

44A

Fig. 4

Bestimmung von markanten Punkten im 3D-Modell mittels 3D-Merkmals-Deskriptoren — 50

Klassifizierung der markanten Punkte und Filterung mittels 3D-Merkmals-Deskriptoren — 51

Koordinatentransformation in das Koordinatensystem der Hauptachsen der Menge der markanten Punkte (PCA) — 52

Lokalisierung von Nasenbrücke und Scharnieren mit Hilfe der klassifizierten markanten Punkte — 53

Bestimmung des Punktes der Nasenrückenauflage für die Grob-Positionierung

54

Segmentierung in die Komponenten: Bügel links, Bügel rechts, Fassung

55

Bestimmung der Auflagekurve an der Bügelunterseite

56

# Fig. 5

Fig. 6A

Fig. 6B

EP 3 657 236 B1

Fig. 7A

Fig. 7B

28

Bestimmung des Anfangspunktes und Endpunktes des Bügelteils und der Bügellänge /⌐ 80

↓

Bestimmung des potenziellen Ohr-Auflagebereichs ⌐ 81

↓

Auflagekurve: Punkte der Unterkante mittels Sliding Window in z-Richtung ⌐ 82

## Fig. 8

Grob-Positionierung an Hand des Aufsetzpunktes und Nasenrückenauflage ⌐ 90

↓

Aufbiegen der Fassung ⌐ 91

↓

Positionierung der Bügel, Bestimmung Rotation um x-Achse ⌐ 92

↓

93 — xy-Fein-Positionierung der Fassung

↓

94 — Bildsynthese der Fassung und des Kopfes

↓

Interaktion des Benutzers mit dem Modell

95

Geste zur Navigation

Geste zur Drehung der Fassung

96 ◇

Geste zur Bewegung auf dem Nasenrücken

## Fig. 9

Fig. 10A

Fig. 10B

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20030123026 A1
- US 2002105530 A1
- WO 2015101738 A2
- US 20160327811 A1
- US 9286715 B2
- US 2016360970 A1
- US 20160246078 A1
- US 20100022304 A1
- US 20150055085 A1
- WO 2013177456 A1

- DE 10216824 A1
- FR 3065295 A1
- US 2013088490 A1
- FR 3016051 A1
- ES 2604806 A2
- WO 0188654 A2
- WO 2016164859 A1
- EP 17153559
- EP 17153556
- EP 17153560

**In der Beschreibung aufgeführte Nicht-Patentliteratur**

- RKEM: Redundant Keypoint Elimination Method in Image Registration. **HOSSEIN-NEJAD ZAHRA et al.** IET Image Processing. IET, 01. Mai 2017, vol. 11, 273-284
- **RAU J-Y ; YEH P-C.** A Semi-Automatic Image-Based Close Range 3D Modeling Pipeline Using a Multi-Camera Configuration. *Sensors,* 2012, vol. 12 (8), 11271-11293
- **M. NIEßNER ; M. ZOLLHÖFER ; S. IZADI ; M. STAMMINGER.** Real-time 3D reconstruction at scale using voxel hashing. *ACM Trans. Graph.,* November 2013, vol. 32 (6
- **CIGNONI, PAOLO et al.** Meshlab: an open-source mesh processing tool. *Eurographics Italian Chapter Conference,* 2008, vol. 2008
- Metadaten. Marz 2017
- **H. HIRSCHMÜLLER.** Stereo Processing by Semiglobal Matching and Mutual Information. *IEEE Transactions on Pattern Analysis and Machine Intelligence,* Februar 2008, vol. 30 (2), 328-341
- Maschinelles Lernen. Marz 2017
- Learning a Descriptor-Specific 3D Keypoint Detector. **SAMUELE SALTI ; FEDERICO TOMBARI ; RICCARDO SPEZIALETTI ; LUIGI DI STEFANO.** Proceedings of the 2015 IEEE International Conference on Computer Vision (ICCV) (ICCV '15). IEEE Computer Society, 2015, 2318-2326

- **R.B. RUSO.** Semantic 3D Object Maps for Everyday Manipulation in Human Living Environments. *Dissertation an der TU München,* 2009, 57
- Überwachtes Lernen. Marz 2017
- Machine Learning Overview. Open Source Computer Vision, Marz 2017
- **BREIMAN, LEO.** Random forests. *Machine learning,* 2001, vol. 45.1, 5-32
- *principal component analysis*
- Spline. Marz 2017
- **DE BOOR, CARL et al.** A practical guide to splines. Springer-Verlag, 1978, vol. 27
- Bildsynthese. Marz 2017
- **V. KAZEMI et al.** *One Millisecond Face Alignment with an Ensemble of Regression Trees*
- **X. ZHU et al.** Face detection, pose estimation and landmark localization in the wild. *CVPR,* 2012
- **RICHARD HARTLEY ; ANDREW ZISSERMAN.** Multiple View Geometry in Computer Vision. Cambridge University Press, 2000
- Ray Tracing. Marz 2017